# EUROPEAN PATENT APPLICATION

(11) **EP 3 586 860 A1**
(43) Date of publication of application: **01.01.2020**
(21) Application number: 18179269.8
(22) Date of filing: 22.06.2018
(51) Int. Cl.: A61K 38/00, C07K 19/00, C07K 14/47, C12N 15/62, A61P 37/02

(54) **COMPLEMENT INHIBITORS AND USES THEREOF**

(71) Applicant: Universität Ulm, 89081 Ulm (DE)
(72) Inventor: SCHMIDT, Christoph, 72589 Westerheim (DE); HUBERT-LANG, Markus, 89134 Blaustein Markbronn (DE)
(74) Representative: Altmann Stößel Dick Patentanwälte PartG mbB

(57) **Abstract**

The present invention relates to a multi-module polypeptide comprising (i) an Fc receptor binding module; (ii) a first complement control protein repeat (CCP) module; and (iii) a second CCP module binding to at least one host cell surface marker, to complement factor C3b, to complement factor C4b, to a degradation product of complement factor C3b, and/or to a degradation product of complement factor C4b; wherein said second CCP module is C-terminal of said Fc receptor binding module and of said first CCP module. The present invention also relates to a polynucleotide encoding said multi-module polypeptide, and to said multi-module polypeptide for use in medicine, in particular for use in treating and/or preventing inappropriate complement activation and/or a disease having inappropriate complement activation as a symptom. Moreover, the present invention relates to an in vitro method for preventing or reducing the degree of complement activation comprising applying a multi-module polypeptide to a reaction mixture, a tissue, and/or an organ comprising complement factors, thereby preventing or reducing the degree of complement activation in said reaction mixture, tissue, and/or organ.

## Description

The present invention relates to a multi-module polypeptide comprising (i) an Fc receptor binding module; (ii) a first complement control protein repeat (CCP) module; and (iii) a second CCP module binding to at least one host cell surface marker, to complement factor C3b, to complement factor C4b, to a degradation product of complement factor C3b, and/or to a degradation product of complement factor C4b; wherein said second CCP module is C-terminal of said Fc receptor binding module and of said first CCP module. The present invention also relates to a polynucleotide encoding said multi-module polypeptide, and to said multi-module polypeptide for use in medicine, in particular for use in treating and/or preventing inappropriate complement activation and/or a disease having inappropriate complement activation as a symptom. Moreover, the present invention relates to an in vitro method for preventing or reducing the degree of complement activation comprising applying a multi-module polypeptide to a reaction mixture, a tissue, and/or an organ comprising complement factors, thereby preventing or reducing the degree of complement activation in said reaction mixture, tissue, and/or organ.

The immune system can be divided in two branches: the phylogenetically older innate immunity and the adaptive immune responses. An immune response by the adaptive, or acquired immune system, is typically more specific than an innate immune response. Other characteristics of the adaptive immune system are the development of an immunological memory and the typically observed delay between exposition of an antigen and the maximal immune response.

The innate immune system is highly conserved even in primitive organisms. The cellular effectors of this branch comprise mainly neutrophils, monocytes and macrophages, whereas the soluble innate immune effectors consist mainly of the complement system in addition to other effectors like acute phase proteins or pore-forming peptides (Parkin & Cohen (2001) The Lancet 357: 1777-89.). The complement system consists of heat-labile components in serum, which were described by Paul Ehrlich to "complement" the antibody response against bacteria. Other functions of the complement system are opsonisation of microbial intruders, immune complexes, debris, apoptotic and necrotic cells to support their effective clearance through uptake by phagocytic cells (Ricklin et al. (2010) Nature Immunology 11: 785-797). The complement system is organized in three activation pathways: the classical (CP), lectin (LP) and alternative pathway (AP).

Activation of the CP is typically achieved in an antibody-dependent manner via the complement component C1q, which acts as a pattern-recognition molecule (PRM). After a series of proteolytic activation events, the CP C3-convertase (C4bC2a) cleaves the complement component C3, which is central to all three complement activation pathways, to C3a, an anaphylatoxin and C3b (opsonin). Due to this cleavage, a conformational change occurs and a previously internal thioester bond reaches the protein surface of C3b. This active, and once it is exposed short lived, thioester bond can bind covalently to hydroxyl- and amino-groups of molecules localized on cell surfaces, or can be lysed ("quenched") by water. As a consequence opsonisation of cells with many C3b molecules can occur if C3-convertases are not down regulated. The production of huge amounts of C3b molecules facilitates activation of C5 by C5 convertases. The C5-convertase cleaves C5 to C5a (the most potent anaphylatoxin) and C5b, which recruits the complement factors C6-9 to form the membrane attack complex (MAC) that assembles holes in cell membranes to lyse and kill.

The Lectin pathway (LP) is similarly organized as the CP. Activation occurs via recognition of pathogen-associated molecular patterns (PAMPs) or danger-associated molecular patterns (DAMPs). Within the LP, PAMPs or DAMPs can be detected by several pattern recognition molecules which are homologous to C1q (the pattern recognition molecule of the CP): mannose-binding lectin (MBL) and various types of collectins and ficolins. Subsequent to PAMP or DAMP, binding MBL undergoes conformational changes and then associates with MBL-associated serine proteases (MASPs). MBL is homologous in structure and function to C1q. In analogy to the CP, MASP2 proteolytically activates C2 into C2a and C2b, and C4 into C4a and C4b. The activated components can build the C3 convertase C4bC2a of the LP, which is identical to the CP and cleaves C3 into C3a and C3b. In analogy to the CP, in absence of strict regulation of the C3 convertase production of more C3b molecules fosters the activation of C5 via C5 convertases. Proteolytic activation of C5 is the starting point of the terminal and lytic complement pathway where C5b initiates formation of MAC.

The alternative pathway gets activated through a process of self-activation at low level. This process is called "tick-over" activation of C3. C3 molecules have an intrinsically metastable conformation. At all times, a small proportion of the C3 molecules undergo spontaneous conformational changes (activation) which exposes the previously internal thioester module. The thioester can be quenched by water or attach indiscriminatingly (for self or foreign) to nucleophiles on a cell surface. Such "auto-activated" C3 is called C3(H₂O) and is structurally similar to C3b molecules. C3b or C3(H₂O) expose new protein surfaces that are hidden in C3. These new surfaces bind Factor B, another complement factor of the AP. When Factor B is bound to C3b or C3(H₂O), it can be cleaved by the protease Factor D into Ba and Bb. Bb remains bound to C3(H₂O) (or C3b) and constitutes the C3 convertase of the AP, C3bBb. In analogy to the CP and LP C3 convertases, C3bBb can produce C3b and C3a molecules by cleaving C3. The protein Properdin, a positive regulator of the AP, plays an important role by stabilizing the protein-protein interactions of the AP C3 convertase. If not regulated, any C3b generated by the alternative, classical or lectin pathway is able to build more C3 convertases of the AP and further amplify the number of produced C3b molecules in positive feedback loop. This step is called "amplification loop" of the AP. Thus, the three pathways of activation converge at the level of C3 activation and, if not regulated, cumulate in MAC formation.

Classical and lectin pathways are inactive until they get specifically activated through the sensing of pathogens or endogenous danger molecules. The AP, on contrary, is active all the time at a low level and indiscriminately produces C3b (or initially C3(H₂O)) molecules. More than ten different regulatory proteins within the complement system are known. Some regulators inhibit right at the level of initiating the CP and LP, however the parts of the cascade that are most tightly controlled are the convertases, which act as amplifiers of the activation signal, and C3b, which builds the platform to form the C3-, and the inflammatory C5-convertases. There are also some regulators that specifically control the lytic MAC.

Regulatory proteins can be divided into decay accelerators which destabilize C3-convertase and lead to faster decay of the convertase. A further group involves proteins which degrade C3b or/and C4b, like Factor H and Factor I; to prevent non-specific degradation by the soluble protease Factor I, inactivation of C3b or C4b necessitates the presence of cofactor proteins that bind to the target and recruit Factor I (e.g. FH or CR1). A further group of regulators inhibits formation of MAC.

Many diseases, in particular hereditary diseases, are associated with a malfunction of complement, in particular overactivation of complement. Thus, in an effort to provide an artificial regulator of the complement system, a monoclonal antibody specifically binding to complement protein C5 and inhibiting terminal activation, eculizumab, was developed (Hillmen et al. (2006), NEJM355(12):1233). In a similar line of development, C5 inhibitory protein rEV576 (coversin) was developed (Romay-Penabad et al (2014), Lupus 23(12):1324). Further, a protein called "mini-FH", connecting complement control protein repeats (CCP domains) 1-4 and 19-20 of complement factor H via a linker was obtained (WO 2013/142362 A1). MiniFH has an increased complement regulatory activity and outperforms FH in its regulatory activity directed towards the complement alternative pathway tenfold in several in vitro and ex vivo assays.

Nonetheless, there is still a need in the art for improved complement inhibitors avoiding the drawbacks of the prior art. This problem is solved by the means and methods disclosed herein.

Accordingly, the present invention relates to a multi-module polypeptide comprising (i) an Fc receptor binding module; (ii) a first complement control protein repeat (CCP) module; and (iii) a second CCP module binding to at least one host cell surface marker, to complement factor C3b, to complement factor C4b, to a degradation product of complement factor C3b, and/or to a degradation product of complement factor C4b; wherein said second CCP module is C-terminal of said Fc-receptor binding module and of said first CCP module.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

As used herein, the term "standard conditions", if not otherwise noted, relates to IUPAC standard ambient temperature and pressure (SATP) conditions, i.e. preferably, a temperature of 25°C and an absolute pressure of 100 kPa; also preferably, standard conditions include a pH of 7. Moreover, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of' means including the components specified but excluding other components except for materials present as impurities, unavoidable materials present as a result of processes used to provide the components, and components added for a purpose other than achieving the technical effect of the invention. For example, a composition defined using the phrase "consisting essentially of' encompasses any known acceptable additive, excipient, diluent, carrier, and the like. Preferably, a composition consisting essentially of a set of components will comprise less than 5% by weight, more preferably less than 3% by weight, even more preferably less than 1%, most preferably less than 0.1% by weight of non-specified component(s). In the context of nucleic acid sequences, the term "essentially identical" indicates a %identity value of at least 80%, preferably at least 90%, more preferably at least 98%, most preferably at least 99%. As will be understood, the term essentially identical includes 100% identity. The aforesaid applies to the term "essentially complementary" mutatis mutandis.

As used herein, the term "Fc receptor" relates to a receptor on the surface of a cell or in endosomes having affinity for the Fc portion of an antibody, preferably of an IgG; thus, preferably, the Fc receptor is an neonatal Fc receptor (FcRn) (or an Fc-gamma receptor), more preferably, the Fc receptor is selected from CD64, CD32, CD16a, and CD16b. Preferably, the Fc receptor is a mammalian Fc receptor, more preferably a human Fc receptor. Accordingly, the term "Fc receptor binding module", as used herein, relates to a module, preferably a polypeptide domain, of the multi-module polypeptide having affinity for an Fc-receptor as specified herein above. Preferably, the dissociation constant K_{D} for the Fc receptor binding module and at least one Fc receptor is at most 10⁻⁶ M, more preferably at most 10⁻⁷ M, even more preferably at most 10⁻⁸ M. Preferably, the Fc receptor is FcRn as specified herein above and the dissociation constant of the FcRn / Fc receptor binding module complex is less than 10⁻⁶ M, preferably less than 10⁻⁷ M at pH 6; also preferably, the Fc receptor is FcRn as specified herein above and the dissociation constant of the FcRn / Fc receptor binding module complex is at least 10⁻⁶ M, preferably at least 10⁻⁵ M at pH 7; thus, preferably, the Fc receptor is FcRn as specified herein above and the dissociation constant of the FcRn / Fc receptor binding module complex is less than 10⁻⁶ M, preferably less than 10⁻⁷ M at pH 6 and at least 10⁻⁶ M, preferably at least 10⁻⁵ M at pH 7. Also preferably, the dissociation constant of the FcRn / Fc receptor binding module complex is at leat 5fold, more preferably at least 10fold, more preferably at least 20fold higher at pH 7 compared to pH 6. Preferably, the Fc receptor binding module is an Fc module of an immunoglobulin (Ig), more preferably of an IgG, still more preferably of an IgG1. Most preferably, the Fc receptor binding module comprises a peptide having an amino acid sequence of SEQ ID NO:3 or a sequence at least 70% identical thereto, preferably having an amino acid sequence of SEQ ID NO:3 or 12.

The term "complement control protein repeat domain", which is also referred to as "complement control protein repeat", "CCP domain", or "CCP" herein and which is also known as "short complement-like repeat", "short consensus repeat" or "SCR" in the art, is, in principle, known in the art; CCP domains were e.g. reviewed in Schmidt et al. (2008), Clin Exp Immunol. 151(1):14-24. CCP domains are peptide sequences comprising approx. 60 to 70 amino acids including a conserved tryptophan and four conserved cysteine residues forming two disulfide bonds, with considerable sequence variation of the residual amino acids. Besides binding to complement proteins C3b and/or C4b, CCP domains were found to mediate further activities, including decay accelerating activity and Factor I cofactor activity as specified herein below.

The term "first CCP module", as used herein, relates to a module of the multi-module polypeptide comprising at least one CCP domain and being a (i) convertase decay accelerating module for convertases of the classical and/or alternative pathways of complement activation; and/or (ii) being a binding module for complement factors C3b and/or C4b, preferably further having Factor I cofactor activity. Thus, preferably, the first CCP module comprises at least one CCP domain having decay accelerating activity on C3 convertases of the alternative and/or the classical pathway of complement activation. The term "decay accelerating activity" as used herein, relates to the property of a CCP domain or CCP module to mediate decay, preferably inactivation, of the C3 convertase of the alternative pathway of complement activation, i.e. C3bBb, and/or of the C3 convertase of the classical pathway of complement activation, i.e. C4bC2a. Preferably, decay accelerating activity of a CCP domain or CCP module is determined by surface plasmon resonance (SPR). Preferably, the first CCP module comprises of from two to ten, more preferably of from two to five, still more preferably of from three to four CCP domains having or contributing to the aforesaid activity. Preferably, the first CCP module comprises CCP domains 1 to 4 of a factor H, preferably human factor H; comprises CCP domains 1 to 3 of a complement receptor type 1 (CR1), preferably of human CR1, as specified herein below; comprises CCP domains 1 to 4 of a decay accelerating factor (DAF), preferably human DAF, as specified herein below; and/or comprises CCP domains 1 to 3 of a C4-binding protein (C4BP), preferably of human C4BP. Preferably, the first CCP-comprising module comprises CCP domains 1 to 3 of a complement receptor type 1 (CR1), as in the naturally occurring sequence; and/or comprises CCP domains 1 to 4 of a decay accelerating factor (DAF), as in the naturally occurring sequence. Preferably, the first CCP module comprises CCP domains 1 to 4 of human factor H. More preferably, the first CCP module comprises, preferably consists of, an amino acid sequence as shown in SEQ ID NO:1 or an amino acid sequence being at least 70%, preferably at least 80%, more preferably at least 90%, most preferably at least 95% identical to SEQ ID NO:1 and having the activity of being a convertase decay accelerating module for convertases of the classical and/or alternative pathways of complement activation. More preferably, the first CCP module comprises, preferably consists of, an amino acid sequence as shown in SEQ ID NO:1.

Also preferably, the first CCP module comprises at least one, preferably at least two, more preferably at least three CCP domains having binding activity for complement factors C3b and/or C4b, preferably further having Factor I cofactor activity. As will be understood by the skilled person, one or more CCP domains having binding activity for complement factors C3b and/or C4b may be CCP domains different from the CCP domains having decay accelerating activity as specified above; preferably, at least one, more preferably at least two, still more preferably at least three CCP domains having binding activity for complement factors C3b and/or C4b are also CCP domains having decay accelerating activity; more preferably, the CCP(s) having binding activity for complement factors C3b and/or C4b are the CCP(s) having decay accelerating activity as specified above. The term "binding activity for complement factors C3b and/or C4b" is understood by the skilled person. Preferably, the term relates to the property of the first CCP module and/or of at least one of its CCP domains to bind to at least one of complement proteins C3b and C4b with measurable affinity, more preferably with a K_{D} of at most 5 x10⁻⁵ M, more preferably at most 1 x 10⁻⁵ M, even more preferably at most 10⁻⁶ M. Preferably, binding affinity of a CCP or a CCP module to C3b or C4b is determined by surface plasmon resonance (SPR). Preferably, at least one CCP having binding activity for complement factors C3b and/or C4b is selected from the list consisting of CCP domains 1 to 4 of a factor H, CCP domains 8 to 10 and 15 to 17 of a CR1, preferably of human CR1, and CCP domains 1-3 of a C4BP, preferably human C4BP. Thus, preferably, the first CCP module comprises or further comprises CCP domains 8 to 10 and/or 15 to 17 of a CR1, preferably of human CR1 as specified elsewhere herein. More preferably, the first CCP-comprising module comprises or further comprises CCP domains 15 to 17 of CR1, preferably of human CR1.

The term "complement receptor type 1" or "CR1" is, in principle, known to the skilled person as relating to a member of the regulators of complement activation (RCA) family of proteins which is also known as C3b/C4b receptor or cluster of differentiation 35 protein (CD35). Preferably, CR1 is a mammalian CR1, more preferably, CR1 is human CR1. Most preferably, CR1 is human CR1 having an amino acid sequence as specified in Genbank Acc. No. P17927.3 GI:290457678.

The term "decay accelerating factor" or "DAF" is, in principle, also known to the skilled person as relating to a cell surface-bound regulator of the complement system which is also known as cluster of differentiation 55 protein (CD55). Preferably, DAF is a mammalian DAF, more preferably human DAF. Most preferably, DAF is human DAF having an amino acid sequence as specified in Genbank Acc. No. P08174.4 GI:60416353.

The term "Factor H" is also known to the skilled person as a cofactor in the inactivation of C3b by factor I and for increasing the rate of dissociation of the C3bBb complex (C3 convertase) and the C3bBb3b complex (C5 convertase) in the alternative complement pathway. Preferably, Factor H is a mammalian Factor H, more preferably human Factor H. Most preferably, Factor H is human Factor H having an amino acid sequence as specified in Genbank Acc. No. NP_000177.2.

The term "C4BP" is also known to the skilled person as a control molecule of the classical pathway of complement activation binding as a cofactor to C3b/C4b for proteolytic inactivation of the complement protein C4b by the serum protease Factor I. C4BP also increases the rate of dissociation of the C4b2a complex (C3 convertase) and the C4b2a3b complex (C5 convertase) in the classical complement pathway. Preferably, C4BP is mammalian C4BP, more preferably human C4BP. Most preferably, C4BP is human C4BP having an amino acid sequence as specified in Genbank Acc. No: NP_000706.1.

The term "second CCP module", as used herein, relates to a module of the multi-module polypeptide having the activity of binding to at least one host cell surface marker, to complement factor C3b, to complement factor C4b, to a degradation product of complement factor C3b, and/or to a degradation product of complement factor C4b. Preferably the second CCP module has the activity of binding to polyanionic carbohydrates comprising sialic acids and/or glycosaminoglycans, and/or the activity of binding to complement factor C3b or C4b, and/or to their degradation products, preferably to iC3b, C3dg, C3d, iC4b, C4dg and/or C4d. Preferably, the second CCP module has binding activity to at least one host cell surface marker. More preferably, the second CCP module has binding activity to at least one host cell surface marker and to at least C3b. Preferably, the second CCP module has no detectable convertase decay accelerating activity and/or has no detectable Factor I cofactor activity. Thus, more preferably, the second CCP module has only binding activity to at least one of the molecules indicated above and, most preferably, is devoid of complement modulating activity. Preferably, the second CCP module comprises at least one, preferably at least two CCP domains having binding activity to host cell surface markers, preferably polyanionic carbohydrates comprising sialic acids and/or glycosaminoglycans and/or having binding activity to complement factor C3b degradation products, preferably to iC3b, C3dg, C3d, iC4b, C4dg and/or C4d.. Preferably, the second CCP module comprises CCP domains 6 to 8 and/or 19 to 20 of a complement Factor H, preferably a human complement Factor H as specified herein above, or any of CCP domains 1-8 of the alpha-chain of C4BP. More preferably, the second CCP module comprises CCP domains 6 to 8 and/or 19 to 20 of a complement Factor H, preferably a human complement Factor H as specified herein above. Even more preferably, the second CCP module comprises, preferably consists of, an amino acid sequence as shown in SEQ ID NO:2 or an amino acid sequence being at least 70%, preferably at least 80%, more preferably at least 90%, most preferably at least 95% identical to SEQ ID NO:2, preferably having binding activity to host cell surface markers, preferably polyanionic carbohydrates comprising sialic acids and/or glycosaminoglycans and/or having binding activity to complement factor C3b degradation products, preferably to iC3b and/or C3dg. More preferably, the second CCP module comprises, preferably consists of, an amino acid sequence as shown in SEQ ID NO:2. Preferably, binding activity to host cell surface markers and binding activity to complement factor C3b degradation products of a CCP or of a host cell recognition module is determined by surface plasmon resonance (SPR).

As used in this specification, the term "multi-module polypeptide" relates to any chemical molecule comprising at least the polypeptide modules as specified herein below. It is to be understood that the chemical linkage between the modules need not necessarily be a peptide bond. It is also envisaged by the present invention that the chemical bond between the modules is an ester bond, a disulfide bond, or any other suitable covalent chemical bond known to the skilled artisan. Also envisaged are non-covalent bonds with a dissociation constant so low that a module will only dissociate to a negligible extent from the other modules. Preferably, the dissociation constant for said non-covalent bond is less than 10⁻⁵ M (as it is the case with the Strep-Tag : Strep-Tactin binding), less than 10⁻⁶ M (as it is the case in the Strep-TagII : Strep-Tactin binding), less than 10⁻⁸ M, less than 10⁻¹⁰ M, or less than 10⁻¹² M (as it is the case for the Streptavidin : Biotin binding). Methods of determining dissociation constants are well known to the skilled artisan and include, e.g., spectroscopic titration methods, surface plasmon resonance measurements, equilibrium dialysis and the like. Moreover, it is also envisaged that the binding between the modules of the multi-module polypeptide is indirect, e.g. that the modules comprise a tag with affinity for biotin and are bound to a further molecule or particle comprising biotin moieties. Preferably, the chemical linkage between the modules is a peptide bond, i.e., preferably, the multi-module polypeptide is a fusion polypeptide comprising or consisting of the modules of the present invention. In a preferred embodiment, the polypeptide consists of the components as described herein.

Preferably, reference to polypeptides, in particular multi-module polypeptides, and/or modules, in particular CCP modules, includes variants of the specific polypeptides and modules described herein. As used herein, the terms "polypeptide variant" and "module variant" relates to any chemical molecule comprising at least the module or modules as specified herein, but differing in structure from said polypeptide or module indicated. Preferably, a polypeptide variant or a module variant comprises a peptide having an amino acid sequence corresponding to an amino acid sequence of from 25 to 500, more preferably of from 30 to 300, most preferably, of from 35 to 150 consecutive amino acids comprised in a polypeptide or module as specified herein. Moreover, it is to be understood that a polypeptide variant or module variant as referred to in accordance with the present invention shall have an amino acid sequence which differs due to at least one amino acid substitution, deletion and/or addition, wherein the amino acid sequence of the variant is still, preferably, at least 50%, 60%, 70%, 80%, 85%, 90%, 92%, 95%, 97%, 98%, or 99% identical with the amino acid sequence of the specific polypeptide or module. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the sequence it is compared to for optimal alignment. The percentage is calculated by determining, preferably over the full length of the peptide, the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Polypeptide variants or module variants referred to above may be derived from allelic variants or any other species specific homologs, paralogs, or orthologs. Moreover, polypeptide variants referred to herein include fragments of the specific polypeptides or the aforementioned types of variants as long as these fragments and/or variants comprise the modules as referred to above. Such fragments may be or be derived from, e.g., degradation products or splice variants of the polypeptides. Further included are variants which differ due to posttranslational modifications such as phosphorylation, glycosylation, ubiquitinylation, sumoylation, or myristylation, by including non-natural amino acids, and/or by being peptidomimetics.

Preferably, at least two modules of the multi-module polypeptide are connected by a "linker" peptide. Suitable linker peptides are, in principle, known in the art. Preferred linker peptides comprise or, preferably, consist of glycine, alanine, and/or proline residues. More preferably, a linker peptide is a poly-glycine linker peptide. Most preferably, a linker peptide, in particular a linker peptide linking a first CCP module and a second CCP module as specified elsewhere herein, is a linker comprising, preferably consisting of, 14 or 15 glycine residues. Other preferred linkers are linkers consisting of 5 or 8 glycine residues. Preferably, it is also envisaged that two modules are connected by exchanging the last amino acid of the N-terminal module and/or the first amino acid of the C-terminal module for a G residue.

As used herein, the term "module comprising an amino acid sequence at least 70% identical to X" relates to a module comprising a variant of a module as specified above having an amino acid sequence at least 70% identical to said module. Preferably, the module comprising an amino acid sequence at least 70% identical to X is a variant of X having the activity of X, more preferably as specified herein.

Thus, preferably, the multi-module polypeptide of the present invention and variants thereof have the activity of being an inhibitor of complement activation, i.e. have the activity of inhibiting the complement reaction, preferably in vitro and/or in vivo. Preferably, the multi-module polypeptide and its variants have the activity of inhibiting at least two, more preferably all three activation pathways of the complement system. More preferably, the multi-module polypeptide and variants thereof have the activity of inhibiting at least the alternative pathway and the classical pathway of complement activation, preferably have the activity of inhibiting at least the alternative pathway, the classical pathway, and the lectin pathway of complement activation.

Preferably, the multi-module polypeptide comprises at least two of its modules, preferably comprises all three of its modules as a contiguous polypeptide sequence, i.e., the multi-module polypeptide preferably is a fusion polypeptide comprising said three modules. Preferably, in principle, the three modules may be comprised in such a fusion polypeptide in any order deemed appropriate by the skilled person, provided that the second CCP module is C-terminal of said Fc receptor binding module and of said first CCP module. Preferably, the multi-module polypeptide comprises the indicated modules in the N-terminal to C-terminal order Fc receptor binding module, first CCP module, and second CCP module. As will be understood, the multi-module polypeptide may preferably comprise further domains and structural elements than those referred to herein. More preferably, the multi-module polypeptide consists of the elements referred to herein. However, preferably, the CCP domain or domains having the activity of binding to at least one host cell surface marker, to complement factor C3b, to complement factor C4b, to a degradation product of complement factor C3b, and/or to a degradation product of complement factor C4b is or are C-terminal of other CCP domains and of the Fc receptor binding module, more preferably, the CCP domain or domains having the activity of binding to at least one host cell surface marker, to complement factor C3b, to complement factor C4b, to a degradation product of complement factor C3b, and/or to a degradation product of complement factor C4b is or are the C-terminal elements of the multi-module polypeptide. Even more preferably, the multi-module polypeptide comprises, preferably consists of an amino acid sequence as shown in SEQ ID NO:8 or 10 or is a variant thereof as specified herein above, wherein, preferably, said variant still has the activity of being an inhibitor of complement activation as specified above. Most preferably, the multi-module polypeptide comprises, preferably consists of, an amino acid sequence as shown in SEQ ID NO:8 or 10.

Advantageously, it was found in the work underlying the present invention that it is important to structure multi-module polypeptides comprising an Fc receptor binding module and cell-binding CCP domains such that the cell-binding CCP domains are at or close to the C-terminus of the resulting molecule. Surprisingly, this placement did not significantly influence the plasma half-life of the multi-module polypeptides, but had a clear impact on the effective concentration/dose.

The definitions made above apply mutatis mutandis to the following. Additional definitions and explanations made further below also apply for all embodiments described in this specification mutatis mutandis.

The present invention further relates to a polynucleotide encoding the multi-module polypeptide of the present invention.

The term "polynucleotide", as used in accordance with the present invention relates to a polynucleotide comprising a nucleic acid sequence which encodes a multi-module polypeptide comprising the modules as specified herein above. A polynucleotide encoding a multi-module polypeptide comprising the aforementioned modules has been obtained in accordance with the present invention by synthesizing a polynucleotide encoding the relevant modules using well known techniques.

Thus, the polynucleotide, preferably, comprises the nucleic acid sequence shown in SEQ ID NO:9 or 11, encoding a polypeptide having an amino acid sequence as shown in SEQ ID NO:8 or 10. It is to be understood that a polypeptide having an amino acid sequence as shown in SEQ ID NOs: 8 or 10 may be also encoded due to the degenerated genetic code by other polynucleotides as well.

Moreover, the term "polynucleotide", as used in accordance with the present invention, further encompasses variants of the aforementioned specific polynucleotides. The polynucleotide variants, preferably, comprise a nucleic acid sequence characterized in that the sequence can be derived from the aforementioned specific nucleic acid sequences shown in SEQ ID NOs: 9 and 11 by at least one nucleotide substitution, addition and/or deletion whereby the variant nucleic acid sequence shall still encode a polypeptide comprising the activities as specified above. Variants include polynucleotides comprising nucleic acid sequences which are at least 70%, at least 75%, at least 80%, at least 85%, at least 90%, at least 95%, at least 98% or at least 99% identical to at least one of the nucleic acid sequences shown in SEQ ID NOs: 9 and 11. Moreover, also encompassed are polynucleotides which comprise nucleic acid sequences encoding amino acid sequences which are at least 70%, at least 75%, at least 80%, at least 85%, at least 90%, at least 95%, at least 98% or at least 99% identical to the amino acid sequences shown in SEQ ID NO: 8 or 10. The percent identity values are, preferably, calculated over the entire amino acid or nucleic acid sequence region.

A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (J. Mol. Evolution., 25, 351-360, 1987, Higgins et al., CABIOS, 5 1989: 151-153) or the programs Gap and BestFit (Needleman and Wunsch (J. Mol. Biol. 48; 443-453 (1970)) and Smith and Waterman (Adv. Appl. Math. 2; 482-489 (1981))), which are part of the GCG software packet (Genetics Computer Group, 575 Science Drive, Madison, Wisconsin, USA 53711 (1991)), are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments. Variants also encompass polynucleotides comprising a nucleic acid sequence which is capable of hybridizing to the aforementioned specific nucleic acid sequences, preferably, under stringent hybridization conditions. These stringent conditions are known to the skilled worker and can be found in Current Protocols in Molecular Biology, John Wiley & Sons, N. Y. (1989), 6.3.1-6.3.6. A preferred example for stringent hybridization conditions are hybridization conditions in 6' sodium chloride/sodium citrate (= SSC) at approximately 45°C, followed by one or more wash steps in 0.2' SSC, 0.1% SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1 to 5 ' SSC (pH 7.2). If organic solvent is present in the abovementioned buffer, for example 50% formamide, the temperature under standard conditions is approximately 42°C. The hybridization conditions for DNA:DNA hybrids are preferably for example 0.1 ' SSC and 20°C to 45°C, preferably between 30°C and 45°C. The hybridization conditions for DNA:RNA hybrids are preferably, for example, 0.1 ' SSC and 30°C to 55°C, preferably between 45°C and 55°C. The abovementioned hybridization temperatures are determined for example for a nucleic acid with approximately 100 bp (= base pairs) in length and a G + C content of 50% in the absence of formamide. The skilled worker knows how to determine the hybridization conditions required by referring to textbooks such as the textbook mentioned above, or the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford. Alternatively, polynucleotide variants are obtainable by PCR-based techniques such as mixed oligonucleotide primer- based amplification of DNA, i.e. using degenerated primers against conserved modules of the polypeptides of the present invention. Conserved modules of the polypeptide of the present invention may be identified by a sequence comparison of the nucleic acid sequence of the polynucleotide or the amino acid sequence of the polypeptide of the present invention with sequences of other CCP domains. As a template, DNA or cDNA from animals, preferably mammals, more preferably humans, may be used.

A polynucleotide comprising a fragment of any of the aforementioned nucleic acid sequences is also encompassed as a polynucleotide of the present invention. The fragment shall encode a polypeptide comprising the modules specified above and which, preferably, still has the activity as specified above. Accordingly, the polypeptide may comprise or consist of the modules of the present invention conferring the said biological activities. A fragment as meant herein, preferably, comprises at least 50, at least 100, at least 250 or at least 500 consecutive nucleotides of the aforementioned nucleic acid sequence or encodes an amino acid sequence comprising at least 20, at least 30, at least 50, at least 80, at least 100 or at least 150 consecutive amino acids of the aforementioned amino acid sequence.

The polynucleotides of the present invention either consist of the aforementioned nucleic acid sequences or comprise the aforementioned nucleic acid sequences. Thus, they may contain further nucleic acid sequences as well. Specifically, the polynucleotides of the present invention may encode fusion proteins wherein one partner of the fusion protein is a multi-module polypeptide being encoded by a nucleic acid sequence recited above. Such fusion proteins may comprise as additional part other polypeptides for monitoring expression (e.g., green, yellow, blue or red fluorescent proteins, alkaline phosphatase and the like) or so called "tags" which may serve as a detectable marker or as an auxiliary measure for purification purposes. Tags for the different purposes are well known in the art and comprise FLAG-tags, 6-histidine-tags, MYC-tags and the like.

The polynucleotide of the present invention shall be provided, preferably, either as an isolated polynucleotide (i.e. isolated from its natural context) or in genetically modified form. The polynucleotide, preferably, is DNA, including cDNA, or is RNA. The term encompasses single as well as double stranded polynucleotides. Moreover, comprised are also chemically modified polynucleotides including naturally occurring modified polynucleotides such as glycosylated or methylated polynucleotides or artificial modified one such as biotinylated polynucleotides.

Thus, preferably, the polynucleotide of the present invention a) is a polynucleotide having at least 70% sequence identity to SEQ ID NO:9, b) encodes a polypeptide having at least 70% sequence identity to SEQ ID NO:8, and/or c) is a polynucleotide capable of hybridizing under stringent conditions stringent conditions to SEQ ID NO:9. More preferably, the polynucleotide a) is a polynucleotide comprising, preferably consisting of the nucleic acid sequence of SEQ ID NO: 9 or 11, and/or b) encodes a polypeptide comprising, preferably consisting of the amino acid sequence of SEQ ID NO: 8 or 10. Preferably, the polynucleotide of the present invention encodes a multi-module polypeptide having an activity as specified above.

The present invention further relates to a vector comprising the polynucleotide of the present invention.

The term "vector", preferably, encompasses phage, plasmid, viral or retroviral vectors as well as artificial chromosomes, such as bacterial or yeast artificial chromosomes. Moreover, the term also relates to targeting constructs which allow for random or site- directed integration of the targeting construct into genomic DNA. Such target constructs, preferably, comprise DNA of sufficient length for either homologous or heterologous recombination as described in detail below. The vector encompassing the polynucleotides of the present invention, preferably, further comprises selectable markers for propagation and/or selection in a host. The vector may be incorporated into a host cell by various techniques well known in the art. For example, a plasmid vector can be introduced in a precipitate such as a calcium phosphate precipitate or rubidium chloride precipitate, or in a complex with a charged lipid or in carbon-based clusters, such as fullerens. Alternatively, a plasmid vector may be introduced by heat shock or electroporation techniques. Should the vector be a virus, it may be packaged in vitro using an appropriate packaging cell line prior to application to host cells. Retroviral vectors may be replication competent or replication defective. In the latter case, viral propagation generally will occur only in complementing host/cells.

More preferably, in the vector of the invention the polynucleotide is operatively linked to expression control sequences allowing expression in prokaryotic and/or eukaryotic cells or isolated fractions thereof. Expression of said polynucleotide comprises transcription of the polynucleotide, preferably into a translatable mRNA. Regulatory elements ensuring expression in eukaryotic cells, preferably mammalian cells, are well known in the art. They, preferably, comprise regulatory sequences ensuring initiation of transcription and, optionally, poly-A signals ensuring termination of transcription and stabilization of the transcript. Additional regulatory elements may include transcriptional as well as translational enhancers. Possible regulatory elements permitting expression in prokaryotic host cells comprise, e.g., the lac, trp or tac promoter in E. coli, and examples for regulatory elements permitting expression in eukaryotic host cells are the AOX1 or GAL1 promoter in yeast or the CMV-, SV40-, RSV-promoter (Rous sarcoma virus), CMV-enhancer, SV40-enhancer or a globin intron in mammalian and other animal cells. Moreover, inducible expression control sequences may be used in an expression vector encompassed by the present invention. Such inducible vectors may comprise tet or lac operator sequences or sequences inducible by heat shock or other environmental factors. Suitable expression control sequences are well known in the art. Beside elements which are responsible for the initiation of transcription such regulatory elements may also comprise transcription termination signals, such as the SV40-poly-A site or the tk-poly-A site, downstream of the polynucleotide. In this context, suitable expression vectors are known in the art such as Okayama-Berg cDNA expression vector pcDV1 (Pharmacia), pBluescript (Stratagene), pCDM8, pRc/CMV, pcDNA1, pcDNA3 (InVitrogene) or pSPORT1 (GIBCO BRL). Preferably, said vector is an expression vector and a gene transfer or targeting vector. Expression vectors derived from viruses such as retroviruses, vaccinia virus, adeno-associated virus, herpes viruses, or bovine papilloma virus, may be used for delivery of the polynucleotides or vector of the invention into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994).

Preferably, the vector is a vector mediating expression of the polynucleotide of the present invention in a host cell. The skilled artisan knows how to select combinations of vectors and host cells for propagation of a vector and/or for expression of a protein encoded by the vector. Furthermore, the present invention relates to a host cell comprising the polynucleotide or the vector of the present invention.

A "host cell", as used herein, relates to a bacterial, archaeal, or eukaryotic cell with the capacity to propagate the vector of the present invention and/or to produce a multi-module polypeptide encoded on the vector or the polynucleotide of the invention. Preferably, the host cell is a bacterial cell from the species Escherichia coli, a lepidopteran, a mouse, rat, or a human cell; more preferably, the cell is a yeast cell, preferably of the genus Pichia, more preferably a Pichia pastoris cell. Preferably, the host cell is a cell cultivated in vitro. In a further preferred embodiment, the host cell is a cell in vivo, preferably a retinal pigment epithelial cell, an endothelial cell within the choroid vasculature, and/or another cell within the retina or the choroidea.

The present invention also relates to a multi-module polypeptide according to the present invention, a polynucleotide according to the present invention, a vector according to the present invention, and/or a host cell according to the present invention for use in medicine. Moreover, the present invention also relates to a multi-module polypeptide according to the present invention, a polynucleotide according to the present invention, a vector according to the present invention, and/or a host cell according to the present invention for treating and/or preventing inappropriate complement activation and/or a disease having inappropriate complement activation as a symptom.

As used herein, the term "inappropriate complement activation" relates to a complement activation which is, in timing and/or amplitude, exceeding the normal level of complement activation under the given circumstances. Thus, preferably, inappropriate complement activation is complement activation exceeding, preferably significantly exceeding, the extent of complement activation of a healthy reference, preferably an apparently healthy subject, under the given circumstances. Preferably, inappropriate complement activation is complement activation causing symptoms of disease in a patient. Symptoms of inappropriate complement activation are known in the art and include hemolysis, macular degeneration, episodic swellings, e.g. in hereditary angioedema, and the like. Preferably, inappropriate complement activation is determined by determining complement factor C3 and/or C4 activity in a sample.

As is known to the skilled person, a variety of diseases is associated and/or caused by inappropriate complement activation. Thus, preferably, the present invention also relates to a multi-module polypeptide according to the present invention, a polynucleotide according to the present invention, or a vector according to the present invention for treating and/or preventing a disease having inappropriate complement activation as a symptom. Preferably, said disease having inappropriate complement activation as a symptom is selected from the list consisting of ischemia reperfusion injury, antibody-mediated graft rejection, posttransplantation thrombotic microangiopathy, autoimmune hemolytic anemia, acute and delayed hemolytic transfusion reaction, cold agglutinine disease, rheumatoid arthritis, aquaporin-4-antibody-positive neuromyelitis optica, CD59-deficiency, C3-Glomerulopathy, atypical hemolytic uremic syndrome, paroxysmal nocturnal hemoglobinuria, and age-related macular degeneration.

The term "treating", as used herein, refers to ameliorating the diseases or disorders referred to herein or the symptoms accompanied therewith, preferably to a significant extent. Said treating as used herein also includes an entire restoration of health with respect to the diseases or disorders referred to herein. It is to be understood that treating as used in accordance with the present invention may not be effective in all subjects to be treated. However, the term shall preferably require that a statistically significant portion of subjects suffering from a disease or disorder referred to herein can be successfully treated. Whether a portion is statistically significant can be determined without further ado by the person skilled in the art using various well known statistic evaluation tools, e.g., determination of confidence intervals, p-value determination, Student's t-test, Mann-Whitney test etc. Preferred confidence intervals are at least 90%, at least 95%, at least 97%, at least 98% or at least 99 %. The p-values are, preferably, 0.1, 0.05, 0.01, 0.005, or 0.0001. Preferably, the treatment shall be effective for at least 60%, at least 70%, at least 80%, or at least 90% of the subjects of a given cohort or population.
The tem "preventing", as used herein, refers to retaining health with respect to the diseases or disorders referred to herein for a certain period of time in a subject. It will be understood that the said period of time is dependent on the amount of the drug compound which has been administered and individual factors of the subject discussed elsewhere in this specification. It is to be also understood that prevention may not be effective in all subjects treated with the compound according to the present invention. However, the term preferably requires that a statistically significant portion of subjects of a cohort or population are effectively prevented from suffering from a disease or disorder referred to herein or its accompanying symptoms. Preferably, a cohort or population of subjects is envisaged in this context which normally, i.e. without preventive measures according to the present invention, would develop a disease or disorder as referred to herein. Whether a portion is statistically significant can be determined without further ado by the person skilled in the art using various well known statistic evaluation tools discussed elsewhere in this specification.

The present invention also relates to a multi-module polypeptide according to the present invention, a polynucleotide according to the present invention, or a vector according to the present invention for treating and/or preventing inappropriate complement activation and/or a disease having inappropriate complement activation as a symptom in combination with a complement protein C5 inhibiting polypeptide, preferably Eculizumab.

The present invention further relates to a complement protein C5 inhibiting polypeptide, preferably Eculizumab, or rEV576 (coversin) for treating and/or preventing inappropriate complement activation and/or a disease having inappropriate complement activation as a symptom in combination with a multi-module polypeptide according to the present invention, a polynucleotide according to the present invention, a vector according to the present invention, and/or a host cell according to the present invention.

The term "complement protein C5" is understood by the skilled person as relating to the protein which is cleaved to yield complement proteins C5a and C5b after activation of the complement pathway. Correspondingly, a "complement protein C5 inhibiting polypeptide" is a polypeptide, preferably an antibody, more preferably a monoclonal antibody, specifically recognizing and inhibiting the complement protein C5. Preferably, the complement protein C5 inhibiting polypeptide is an antibody specifically binding to C5 and inhibiting terminal activation; more preferably, the complement protein C5 inhibiting polypeptide is Eculizumab (CAS NO: 219685-50-4). Also preferably, complement protein C5 inhibiting polypeptide is rEV576 (coversin).

The present invention further relates to a combined preparation for simultaneous, separate or sequential use comprising (i) a multi-module polypeptide according to the present invention and (ii) a complement protein C5 inhibiting polypeptide, preferably Eculizumab, or rEV576 (coversin).

The term "combined preparation", as referred to in this application, relates to a preparation comprising the pharmaceutically active compounds of the present invention in one preparation. Preferably, the combined preparation is comprised in a container, i.e. preferably, said container comprises all pharmaceutically active compounds of the present invention. Preferably, said container comprises the pharmaceutically active compounds of the present invention as separate formulations, i.e. preferably, one formulation of the multi-module polypeptide and one formulation of the complement protein C5 inhibiting polypeptide. As will be understood by the skilled person, the term "formulation" relates to a, preferably pharmaceutically acceptable, mixture of compounds, comprising or consisting of at least one pharmaceutically active compound of the present invention. Preferably, the combined preparation comprises a complement protein C5 inhibiting polypeptide and a multi-module polypeptide in a single solid pharmaceutical form, e.g. a tablet, wherein, more preferably, one compound of the present invention is comprised in an immediate or fast release formulation, and the second compound of the present invention is comprised in a slow or retarded release formulation; more preferably, the compounds of the present invention are comprised in two separate, preferably liquid, formulations; said separate liquid formulations, preferably are for injection, preferably at different parts of the body of a subject.

Preferably, the combined preparation is for separate or for combined administration. "Separate administration", as used herein, relates to an administration wherein at least two of the pharmaceutically active compounds of the present invention are administered via different routes and/or at different parts of the body of a subject. E.g. one compound may be administered by enteral administration (e.g. orally), whereas a second compound is administered by parenteral administration (e.g. intravenously). Preferably, the combined preparation for separate administration comprises at least two physically separated preparations for separate administration, wherein each preparation contains at least one pharmaceutically active compound; said alternative is preferred e.g. in cases where the pharmaceutically active compounds of the combined preparation have to be administered by different routes, e.g. parenterally and orally, due to their chemical or physiological properties. Conversely, "combined administration" relates to an administration wherein the pharmaceutically active compounds of the present invention are administered via the same route, e.g. orally or, preferably, intravenously.

Also preferably, the combined preparation is for simultaneous or for sequential administration. "Simultaneous administration", as used herein, relates to an administration wherein the pharmaceutically active compounds of the present invention are administered at the same time, i.e., preferably, administration of the pharmaceutically active compounds starts within a time interval of less than 15 minutes, more preferably, within a time interval of less than 5 minutes. Most preferably, administration of the pharmaceutically active compounds starts at the same time, e.g. by swallowing a tablet comprising the pharmaceutically active compounds, or by swallowing a tablet comprising one of the pharmaceutically active compounds and simultaneous injection of the second compound, or by applying an intravenous injection of a solution comprising one pharmaceutically active compound and injecting second compound in different part of the body. Conversely, "sequential administration, as used herein, relates to an administration causing plasma concentrations of the pharmaceutically active compounds in a subject enabling the synergistic effect of the present invention, but which, preferably, is not a simultaneous administration as specified herein above. Preferably, sequential administration is an administration wherein administration of the pharmaceutically active compounds, preferably all pharmaceutically active compounds, starts within a time interval of 1 or 2 days, more preferably within a time interval of 12 hours, still more preferably within a time interval of 4 hours, even more preferably within a time interval of one hour, most preferably within a time interval of 5 minutes.

Preferably, the combined preparation is a pharmaceutically compatible combined preparation. The terms "pharmaceutically compatible preparation" and "pharmaceutical composition", as used herein, relate to compositions comprising the compounds of the present invention and optionally one or more pharmaceutically acceptable carrier. The compounds of the present invention can be formulated as pharmaceutically acceptable salts. Preferred acceptable salts are acetate, methylester, HC1, sulfate, chloride and the like. The pharmaceutical compositions are, preferably, administered topically or, more preferably, systemically. Suitable routes of administration conventionally used for drug administration are oral, intravenous, subcutaneous, or parenteral administration as well as inhalation. However, depending on the nature and mode of action of a compound, the pharmaceutical compositions may be administered by other routes as well. Moreover, the compounds can be administered in combination with other drugs either in a common pharmaceutical composition or as separated pharmaceutical compositions as specified elsewhere herein, wherein said separated pharmaceutical compositions may be provided in form of a kit of parts. Preferably, the combined preparation is an extended release preparation with regard to one or more of the compounds.

The compounds are, preferably, administered in conventional dosage forms prepared by combining the drugs with standard pharmaceutical carriers according to conventional procedures. These procedures may involve mixing, granulating and compressing or dissolving the ingredients as appropriate for the desired preparation. It will be appreciated that the form and character of the pharmaceutically acceptable carrier or diluent is dictated by the amount of active ingredient with which it is to be combined, the route of administration and other well-known variables.

The carrier(s) must be acceptable in the sense of being compatible with the other ingredients of the formulation and being not deleterious to the recipient thereof. The pharmaceutical carrier employed may be, for example, a solid, a gel or a liquid. Exemplary of solid carriers are lactose, terra alba, sucrose, talc, gelatin, agar, pectin, acacia, magnesium stearate, stearic acid, and the like. Exemplary liquid carriers are phosphate buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania.

The diluent(s) is/are selected so as not to affect the biological activity of the compound or compounds. Examples of such diluents are distilled water, physiological saline, Ringer's solutions, dextrose solution, and Hank's solution. In addition, the pharmaceutical composition or formulation may also include other carriers, adjuvants, or nontoxic, nontherapeutic, nonimmunogenic stabilizers, reactive oxygen scavengers, and the like.

A therapeutically effective dose refers to an amount of the compounds to be used in a pharmaceutical composition of the present invention which prevents, ameliorates or treats the symptoms accompanying a disease or condition referred to in this specification. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

The dosage regimen will be determined by the attending physician and other clinical factors; preferably in accordance with any one of the above described methods. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment. A typical dose can be, for example, in the range of from 1 to 1500 mg; however, doses below or above this exemplary range are envisioned, especially considering the aforementioned factors. Generally, the regimen as a regular administration of the pharmaceutical composition should be in the range of 100 µg to 100 mg units per day. If the regimen is a continuous infusion, it should also be in the range of 100 µg to 100 mg units per kilogram of body weight per minute, respectively. Preferably, extended release preparations of each drug are injected from once per 1 week to once per 2 months or even at longer intervals. Progress can be monitored by periodic assessment. Preferred doses and concentrations of the compounds of the present invention are specified elsewhere herein.

By means of example, a plasma concentration of the multi-module polypeptide preferably is not less than 25 nM, more preferably not less than 50 nM. Also preferably, a plasma concentration of the multi-module polypeptide is in the range of from 20 nM to 20 µM, more preferably of from 50 nM to 5 µM. Effective concentrations of a complement protein C5 inhibiting polypeptide, in particular Eculizumab, are known in the art. Due to the synergistic effect of the multi-module polypeptides of the present invention, the effective concentrations for a complement protein C5 inhibiting polypeptide in combined treatment may be lower.

The pharmaceutical compositions and formulations referred to herein are, preferably, administered at least once, e.g. in case of extended release formulations, in order to treat or ameliorate or prevent a disease or condition recited in this specification. However, the said pharmaceutical compositions may be administered more than one time, for example from one to four times daily up to a non-limited number of days. Also some compounds with a short clearance time may be applied as infusion in blood stream to provide effective dose in whole body during long treatment time.

Specific pharmaceutical compositions are prepared in a manner well known in the pharmaceutical art and comprise at least one active compound referred to herein above in admixture or otherwise associated with a pharmaceutically acceptable carrier or diluent. For making those specific pharmaceutical compositions, the active compound(s) will usually be mixed with a carrier or the diluent, or enclosed or encapsulated in a capsule, sachet, cachet, paper or other suitable containers or vehicles. The resulting formulations are to be adopted to the mode of administration, i.e. in the forms of tablets, capsules, suppositories, solutions, suspensions or the like. Dosage recommendations shall be indicated in the prescribers or users instructions in order to anticipate dose adjustments depending on the considered recipient.

The present invention also relates to a medicament comprising (i) a multi-module polypeptide, (ii) a complement protein C5 inhibiting polypeptide, and (iii) at least one pharmaceutically acceptable carrier; and to said medicament for use in treatment and/or prevention as specified above.

The term "medicament" is understood by the skilled person. As will be understood, the definitions given herein above for the term "combined preparation", preferably, apply to the term medicament of the present invention mutatis mutandis.

Further, the present invention relates to a method for treating and/or preventing inappropriate complement activation and/or a disease having inappropriate complement activation as a symptom in a subject comprising administering an effective dose of a multi-module polypeptide according to the present invention, a polynucleotide according to the present invention, a vector according to the present invention, and/or a host cell according to the present invention to said subject, thereby treating and/or preventing inappropriate complement activation and/or a disease having inappropriate complement activation as a symptom in said subject.

The method for treating and/or preventing of the present invention, preferably, is an in vivo method. Moreover, it may comprise steps in addition to those explicitly mentioned above. For example, further steps may relate, e.g., to diagnosing inappropriate complement activation and/or a disease having inappropriate complement activation as a symptom, or administering additional compounds, e.g. a complement protein C5 inhibiting polypeptide. Moreover, one or more of said steps may be performed by automated equipment.

The term "subject", as used herein, relates to an animal having a complement system, preferably to a mammal. More preferably, the subject is cattle, a pig, sheep, horse, cat, dog, mouse, or rat, most preferably a human.

Moreover, the present invention relates to an in vitro method for preventing or reducing the degree of complement activation comprising applying a multi-module polypeptide according to the present invention, a polynucleotide according to the present invention, a vector according to the present invention, and/or a host cell according to the present invention to a reaction mixture, a tissue and/or an organ comprising complement factors, thereby preventing or reducing the degree of complement activation in said reaction mixture, tissue, and/or organ.

The in vitro method for preventing or reducing the degree of complement activation of the present invention may comprise steps in addition to those explicitly mentioned above. For example, further steps may relate, e.g., to introducing the polynucleotide or the vector of the present invention into a host cell, or determining the degree of complement activation in said reaction mixture, tissue, or organ. Moreover, one or more of said steps may be performed by automated equipment. Preferably, the method is performed on a reaction mixture in vitro.

Further, the present invention relates to a use of a multi-module polypeptide according to the present invention, a polynucleotide according to the present invention, a vector according to the present invention and/or a host cell according to the present invention for treating and/or preventing inappropriate complement activation and/or a disease having inappropriate complement activation as a symptom; and to a use of a multi-module polypeptide according to the present invention, a polynucleotide according to the present invention, a vector according to the present invention, and/or a host cell according to the present invention for manufacturing a medicament for treating and/or preventing inappropriate complement activation and/or a disease having inappropriate complement activation as a symptom.

In view of the above, the following embodiments are preferred:
1. A multi-module polypeptide comprising
   (i) an Fc receptor binding module;
   (ii) a first complement control protein repeat (CCP) module; and
   (iii) a second CCP module binding to at least one host cell surface marker, to complement factor C3b, to complement factor C4b, to a degradation product of complement factor C3b, and/or to a degradation product of complement factor C4b;
   wherein said second CCP module is C-terminal of said Fc receptor binding module and of said first CCP module.
2. The multi-module polypeptide of embodiment 1, wherein said multi-module polypeptide comprises said modules in the N-terminal to C-terminal order Fc receptor binding module, first CCP module, and second CCP module.
3. The multi-module polypeptide of embodiment 1 or 2, wherein said first CCP module and/second CCP module comprises a multitude of CCP domains.
4. The multi-module polypeptide of any one of embodiments 1 to 3, wherein said first and/or second CCP module comprises of from two to ten, preferably of from two to five, more preferably of from three to four CCP domains.
5. The multi-module polypeptide of any one of embodiments 1 to 4, wherein said first CCP module comprises CCP domains 1 to 4 of a factor H, preferably human factor H; comprises CCP domains 1 to 3 of a complement receptor type 1 (CR1), preferably of human CR1; comprises CCP domains 1 to 4 of a decay accelerating factor (DAF), preferably human DAF, and/or comprises CCP domains 1 to 3 of a C4-binding protein (C4BP), preferably of human C4BP.
6. The multi-module polypeptide of any one of embodiments 1 to 5, wherein said first CCP module (i) is a convertase decay accelerating module for convertases of the classical and/or alternative pathways of complement activation and/or (ii) is a binding module for complement factors C3b and/or C4b, preferably further having Factor I cofactor activity.
7. The multi-module polypeptide of any one of embodiments 1 to 6, wherein said first CCP module comprises, preferably consists of, an amino acid sequence as shown in SEQ ID NO:1 or an amino acid sequence being at least 70% identical thereto.
8. The multi-module polypeptide of any one of embodiments 1 to 7, wherein said first CCP module comprises, preferably consists of, an amino acid sequence as shown in SEQ ID NO:1.
9. The multi-module polypeptide of any one of embodiments 1 to 8, wherein said second CCP module binds to polyanionic carbohydrates comprising sialic acids, glycosaminoglycans, and/or complement factor C3b or its degradation products.
10. The multi-module polypeptide of any one of embodiments 1 to 9, wherein said second CCP module comprises CCP domains 19 to 20 of factor H, preferably human factor H.
11. The multi-module polypeptide of any one of embodiments 1 to 10, wherein said second CCP module comprises, preferably consists of, an amino acid sequence as shown in SEQ ID NO:2 or an amino acid sequence being at least 70% identical thereto.
12. The multi-module polypeptide of any one of embodiments 1 to 11, wherein said second CCP module comprises, preferably consists of, an amino acid sequence as shown in SEQ ID NO:2.
13. The multi-module polypeptide of any one of embodiments 1 to 12, wherein said Fc receptor binding module is an Fc module of an immunoglobulin (Ig).
14. The multi-module polypeptide of any one of embodiments 1 to 13, wherein said Fc receptor binding module is an Fc module of an IgG, preferably of an IgG1.
15. The multi-module polypeptide of any one of embodiments 1 to 14, wherein said Fc receptor binding module comprises a peptide having an amino acid sequence of SEQ ID NO:3 or a sequence at least 70% identical thereto, preferably having an amino acid sequence of SEQ ID NO:3.
16. The multi-module polypeptide of any one of embodiments 1 to 15, wherein said multi-module polypeptide comprises at least two of said modules as a contiguous polypeptide sequence, preferably comprises all three of said modules as a contiguous polypeptide sequence.
17. The multi-module polypeptide of any one of embodiments 1 to 16, wherein said first CCP module and/or said second CCP module comprises CCP domains 1 to 4 of a factor H, preferably human factor H;
18. The multi-module polypeptide of any one of embodiments 1 to 17, wherein said first CCP module and said second CCP module together are comprised as a mini-FH comprising the amino acid sequence of one of SEQ ID NO:4, 5, 6, or 7 or a sequence at least 70% identical to at least one of said sequences, preferably comprising the amino acid sequence of one of SEQ ID NO:4, 5, 6, or 7.
19. The multi-module polypeptide of any one of embodiments 1 to 18, wherein at least two of said modules are connected by a linker peptide, preferably a poly-AG or poly-G linker peptide.
20. The multi-module polypeptide of any one of embodiments 1 to 19, wherein said first CCP module and said second CCP module are connected by a linker comprising, preferably consisting of, from 1 to 15, preferably 1, 5, 8, 14, or 15 glycine residues.
21. The multi-module polypeptide of any one of embodiments 1 to 20, wherein said multi-module polypeptide comprises, preferably consists of, an amino acid sequence as shown in SEQ ID NO:8 or an amino acid sequence being at least 70% identical thereto.
22. The multi-module polypeptide of any one of embodiments 1 to 21, wherein said multi-module polypeptide comprises, preferably consists of, an amino acid sequence as shown in SEQ ID NO:8.
23. The multi-module polypeptide of any one of embodiments 1 to 22, wherein said first CCP
   module comprises at least one, preferably at least two, more preferably at least three CCPs having binding activity for complement factors C3b and/or C4b, having Factor I cofactor activity.
24. The multi-module polypeptide of embodiment 23, wherein said CCPs having binding activity for complement factors C3b and/or C4b, have Factor I cofactor activity.
25. The multi-module polypeptide of any one of embodiments 1 to 24, wherein said multi-module polypeptide has the activity of inhibiting the complement reaction.
26. The multi-module polypeptide of any one of embodiments 1 to 25, wherein said multi-module polypeptide has the activity of inhibiting at least one, preferably two, more preferably all three activation pathways of the complement system.
27. The multi-module polypeptide of any one of embodiments 1 to 26, wherein said multi-module polypeptide has the activity of inhibiting at least the alternative pathway and the classical pathway of complement activation, preferably has the activity of inhibiting at least the alternative pathway, the classical pathway, and the lectin pathway of complement activation.
28. A polynucleotide encoding a multi-module polypeptide of any one of embodiments 1 to 27.
29. The polynucleotide of embodiment 28, wherein said polynucleotide
   a) is a polynucleotide having at least 70% sequence identity to SEQ ID NO:9,
   b) encodes a polypeptide having at least 70% sequence identity to SEQ ID NO:8, and/or
   c) is a polynucleotide capable of hybridizing under stringent conditions to SEQ ID NO:9.
30. The polynucleotide of embodiment 28 or 29, wherein said polynucleotide
   a) is a polynucleotide comprising, preferably consisting of the nucleic acid sequence of SEQ ID NO:9 or 11 and/or
   b) encodes a polypeptide comprising, preferably consisting of the amino acid sequence of SEQ ID NO:8 or 10.
31. A vector comprising the polynucleotide of any one of embodiments 28 to 30.
32. A host cell comprising the polynucleotide of any one of embodiments 28 to 30 and/or the vector of embodiment 31.
33. A multi-module polypeptide according to any one of embodiments 1 to 27, a polynucleotide according to any one of embodiments 28 to 30, a vector according to embodiment 31, and/or a host cell according to embodiment 32, for use in medicine.
34. A multi-module polypeptide according to any one of embodiments 1 to 27, a polynucleotide according to any one of embodiments 28 to 30, a vector according to embodiment 31, and/or a host cell according to embodiment 32, for treating and/or preventing inappropriate complement activation and/or a disease having inappropriate complement activation as a symptom.
35. A multi-module polypeptide according to any one of embodiments 1 to 27, a polynucleotide according to any one of embodiments 28 to 30, a vector according to embodiment 31, or a host cell according to embodiment 32, for treating and/or preventing ischemia reperfusion injury, antibody-mediated graft rejection, posttransplantation thrombotic microangiopathy, autoimmune hemolytic anemia, acute and delayed hemolytic transfusion reaction, cold agglutinin disease, rheumatoid arthritis, aquaporin-4-antibody-positive neuromyelitis optica, CD59-deficiency, C3-Glomerulopathy, atypical or typical hemolytic uremic syndrome, paroxysmal nocturnal hemoglobinuria, and/or age-related macular degeneration.
36. A multi-module polypeptide according to any one of embodiments 1 to 27, a polynucleotide according to any one of embodiments 28 to 30, a vector according to embodiment 31, or a host cell according to embodiment 32, for use in treating and/or preventing inappropriate complement activation and/or a disease having inappropriate complement activation as a symptom in combination with a complement protein C5 inhibiting polypeptide, preferably Eculizumab or rEV576 (coversin).
37. A complement protein C5 inhibiting polypeptide, preferably Eculizumab for use in treating and/or preventing inappropriate complement activation and/or a disease having inappropriate complement activation as a symptom in combination with a multi-module polypeptide according to any one of embodiments 1 to 27, a polynucleotide according to any one of embodiments 28 to 30, a vector according to embodiment 31, or a host cell according to embodiment 32.
38. A combined preparation for simultaneous, separate or sequential use comprising (i) a multi-module polypeptide according to any one of embodiments 1 to 27, a polynucleotide according to any one of embodiments 28 to 30, a vector according to embodiment 31, or a host cell according to embodiment 32 and (ii) a complement protein C5 inhibiting polypeptide, preferably Eculizumab or rEV576 (coversin).
39. A method for treating and/or preventing inappropriate complement activation and/or a disease having inappropriate complement activation as a symptom in a subject comprising administering an effective dose of a multi-module polypeptide according to any one of embodiments 1 to 27, a polynucleotide according to any one of embodiments 28 to 30, a vector according to embodiment 31, and/or a host cell according to embodiment 32 to said subject, thereby treating and/or preventing inappropriate complement activation and/or a disease having inappropriate complement activation as a symptom in said subject.
40. An in vitro method for preventing or reducing the degree of complement activation comprising applying a multi-module polypeptide according to any one of embodiments 1 to 28 to a reaction mixture, a tissue, and/or an organ comprising complement factors, thereby preventing or reducing the degree of complement activation in said reaction mixture, tissue, and/or organ.
41. Use of a multi-module polypeptide according to any one of embodiments 1 to 27, a polynucleotide according to any one of embodiments 28 to 30, a vector according to embodiment 31, and/or a host cell according to embodiment 32 for treating and/or preventing inappropriate complement activation and/or a disease having inappropriate complement activation as a symptom.
42. Use of a multi-module polypeptide according to any one of embodiments 1 to 27, a polynucleotide according to any one of embodiments 28 to 30, a vector according to embodiment 31, and/or a host cell according to embodiment 32 for manufacturing a medicament for treating and/or preventing inappropriate complement activation and/or a disease having inappropriate complement activation as a symptom.

All references cited in this specification are herewith incorporated by reference with respect to their entire disclosure content and the disclosure content specifically mentioned in this specification.

### Figure Legends

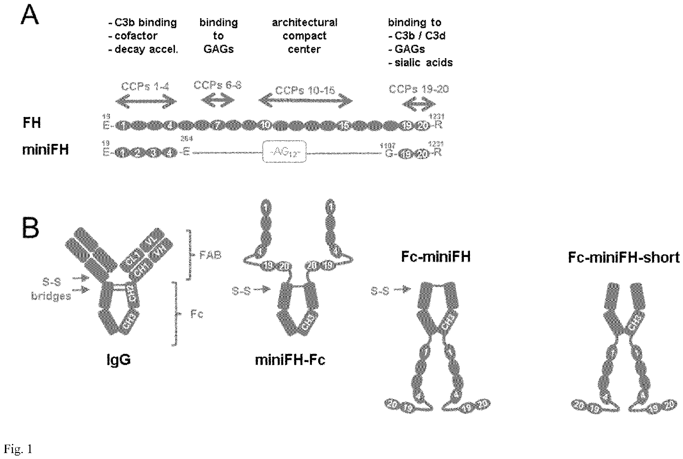

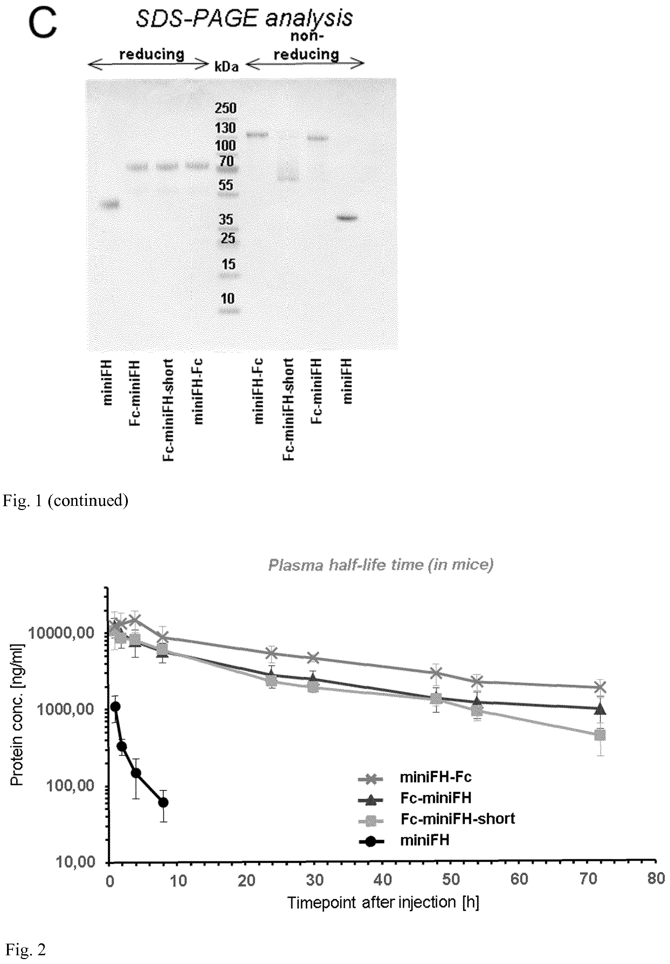

Fig. 1: Natural, engineered and Fc-fused constructs. (A) Schematic domain representation of the natural complement regulator Factor H (FH) and the previously engineered FH variant miniFH. Numbering of amino acids based on encoded FH sequence (UniProt accession number: P08603) including signal sequence. Each oval represents a CCP domain (domain numbers are indicated). Native N-and C-terminal residues are denoted in one letter code; non-native linker sequences are boxed. Key functional properties of CCP domains are highlighted at the top. (B) Schematics of the domain architecture of an IgG molecule and the three miniFH Fc-fusion molecules. Note that only inter-polypeptide chain disulfide bonds (S-S-bridges) are depicted and highlighted by arrows. Inter-chain disulfide bridges exist between the constant domains of the heavy and light chains of an IgG, between the heavy and heavy chain of an IgG, of miniFH-Fc and Fc-miniFH. Internal, i.e. intra-polypeptide chain disulfide bonds are not depicted or highlighted. (C) SDS-PAGE gel analysis of miniFH and the three different Fc-fusion variants of miniFH. 2µg of each protein were loaded onto a Novex NuPAGE 4-12% Bis-Tris SDS-PAGE gel under reducing and non-reducing conditions and visualized by coomassie staining
Fig. 2: Determination of the plasma half-life time of miniFH and the three Fc-fusion variants in mice. Mean plasma circulatory levels of miniFH or the three miniFH Fc-fusion variant. The plasma levels of the proteins were measured by ELISA in plasma prepared from blood collected at indicated times following injection (0.1 mg). Mean ± SD; miniFH n = 3, Fc-miniFH-short n= 5, all others n= 4.

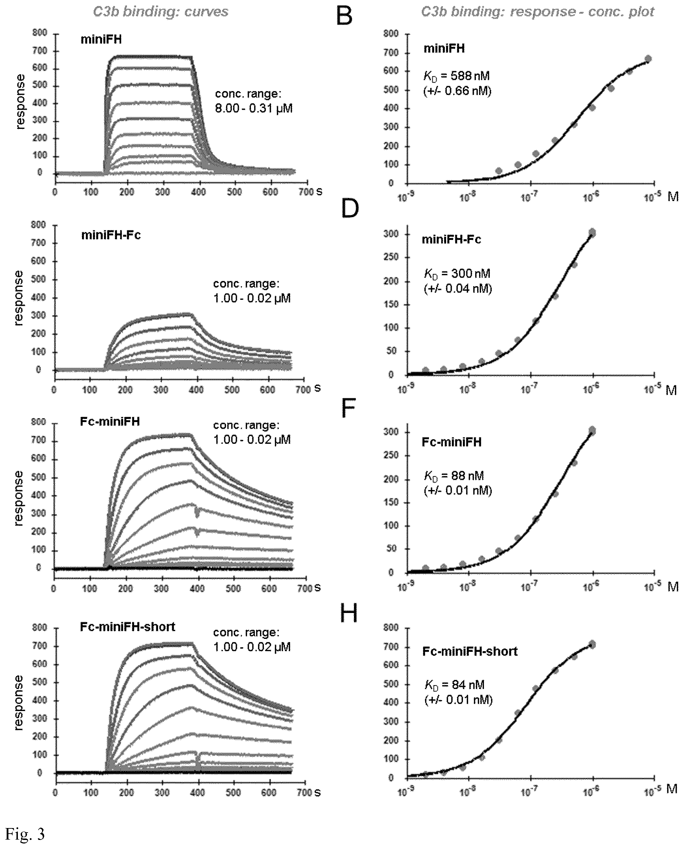

Fig. 3: C3b binding activity of miniFH and Fc-fusion variants of miniFH. Surface plasmon resonance (SPR) sensorgrams for C3b binding of miniFH (A), miniFH-Fc (C), Fc-miniFH (E) and Fc-miniFH-short (G) (assayed at a concentration range as specified; 4030 RU of C3b were deposited by amine). (B) Respective concentration response plots with fitted affinity (1:1 steady-state affinity fit) for C3b binding of miniFH (B), miniFH-Fc (D), Fc-miniFH (F) and Fc-miniFH-short (H) are shown. The affinity equilibrium constant is stated in the panes.

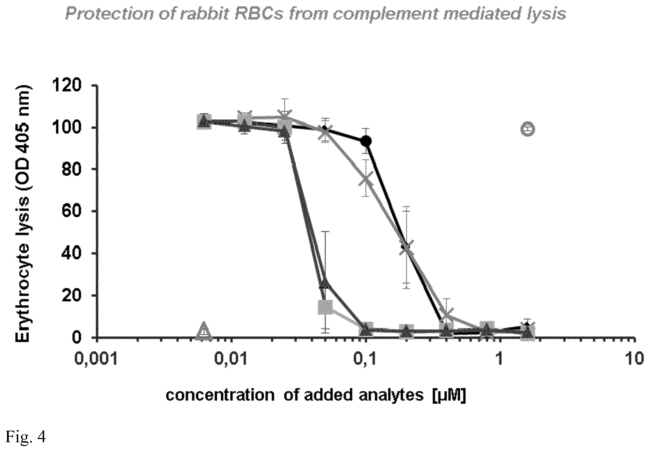

Fig. 4: Protection of rabbit erythrocytes from complement alternative pathway mediated lysis. Rabbit erythrocytes were incubated for 30 min in serum (from healthy donors) that had been mixed with one of the complement inhibitors as indicated. The final serum conc. in the assay was 25%. The lysis of rabbit erythrocytes was measured by hemoglobin release and normalized to lysis observed in water (Average of 3 independent assays with SD is shown).

The following Examples shall merely illustrate the invention. They shall not be construed, whatsoever, to limit the scope of the invention.

### Example 1: Recombinant proteins and FH

The naturally occurring (full-length) plasma protein Factor H (FH) was purified from plasma and was bought from the commercial source CompTech (Tyler, USA). The recombinant proteins miniFH, and the three different Fc-fusions of miniFH (SEQ ID NO:4), miniFH-Fc (SEQ ID NO:13), Fc-miniFH (SEQ ID NO:10), and Fc-miniFH-short (SEQ ID NO:8) were produced and purified in a procedure as previously described (Schmidt et al. (2013), J. Immunol. 190(11):5712-5721). In order to construct the DNA constructs for different Fc:miniFH fusion proteins, the codon-optimised (for the host Pichia pastors) miniFH-encoding DNA and the codon-optimised (for the host Pichia pastors) coding DNA for the Fc part were cloned into the pPICZaB Pichia pastoris expression vector (Invitrogen). Codon optimized DNA was obtained from GeneArt. Utilising specifically introduced restriction enzyme sites (PstI, XmaI and XbaI) the DNA encoding the Fc part and miniFH were digested and ligated together into the pPICZaB Pichia pastoris expression vector in a fashion to produce the desired orientation (see Fig. 1 for construct overview). Transformation of the expression cassettes into the Pichia pastoris strains KM71H or GS115 (Invitrogen) was performed according to the manufacturer's instructions and expressed in P. pastoris using a fermenter according to a procedure (with small variations) as described (Schmidt et al. (2011), Protein Expr. Purif. ;76(2):254-263). The proteins were purified by consecutive cation- and/or anion-exchange chromatography steps, followed by size exclusion chromatography. The native amino acid sequences of all recombinant constructs are expected to be preceded by the non-native sequence EAEAAG (SEQ ID NO:14), EAAG (SEQ ID NO:15) or AG (SEQ ID NO:15), with the EA sequences being the remnants of the processing of the yeast secretion signal peptide (Cereghino et al. (2000), FEMS Microbiol. Rev. 24(1):45-66), and AG being a cloning artefact.

### Example 3: Alternative pathway rabbit erythrocyte hemolysis assay

The assay was performed, with small variation, as published before (Schmidt et al. (2016), Immunobiology. 221(4):503-511). In brief, 20µl of complement inhibitors in PBS were mixed with 10µl NHS (CompTech) containing Mg-EGTA (5mM final assay concentration). 10µl of rabbit erythrocytes (rRBC) in suspension were added and the mix was incubated for 30min at 37°C. To stop the reaction, 120µl of ice-cold PBS/EDTA (5mM) were added. rRBC lysis was quantified by measuring the A405 of 100µl of the supernatant.

### Example 4: Binding of Complement Fc-fusion variants to C3b monitored by surface plasmon resonance (SPR)

Surface plasmon resonance experiments were performed at 25°C on a Reichert SR7500DC SPR instrument in PBS containing 0.005% Tween20 at a flow of 25 µl/min. 4030 response units (RU) of C3b were immobilized onto a carboxymethyldextran (CMD) 500 sensor chip (Xantec) and binding to amine coupled C3b was assayed. The analytes were injected at the indicated concentrations. To probe binding, a concentration series of miniFH or Fc fusions of miniFH were injected (at 25 µl/min for 2.5 min) followed by buffer flow for 300 s and a regeneration step consisting of an injection of 1 M NaCl for 30 s. The highest concentration of each series was injected twice to assess reproducibility. Where appropriate, affinity constants were extracted by plotting the response at steady state against the molar concentration and subsequently fitting the affinity with TRACEDRAWER software using a 1:1 steady-state affinity model. Reference-subtracted sensorgrams are shown throughout.

### Example 5: Determination of plasma half life time in mice

BALB/c mice were injected i.v. with 0.1 mg of analyte protein in sterile PBS. Proteins analytes investigated were cleaned prior to application from endotoxins if necessary, so the doses did not exceed 5 EU endotoxins/kg body weight. Typically, blood was removed from the tail 1, 2, 4, 8, 24, 30, 48, 54 and 72 h after injection of the protein analyte and mixed with the same volume of PBS containing 5mM EDTA to stop the clotting reaction. Plasma was prepared by spinning the EDTA-blood mix for 3 min at 2000-3000g. Plasma was shock frozen in liquid nitrogen and stored at -80°C prior to use in a sandwich ELISA for determining the level of analyte protein in mouse plasma at each time point.

In order to determine miniFH or one of the Fc fusion versions of miniFH in mouse plasma, microtitre plates (MaxiSorp; Nunc) were coated with 2µg/ml of capture antibody (anti-human complement factor H (clone: C18/3), stock: 1mg/ml) with 50µl/well in PBS for 2h at room temperature or overnight at 4°C. After washing twice with 200µl/well PBST (PBS + 0.05% Tween), the wells were blocked with 1% BSA in PBS (= BSA-PBS) with 200µl/well for 1h at RT. Then wells were exposed to dilutions of either the murine plasma samples or the samples of the standard curve of the respective analyte which were prepared by mixing the analyte at set concentrations with the plasma of an untreated mouse. The samples were incubated in the wells for 30 min at RT followed by washing 3 times with PBST (200µl). Then 50µl of goat anti-human Factor H polyclonal antibody (unconjugated, stock: 1.0mg/ml, cat#A237, CompTech) was added at a 1:1000 solution in PBS-BSA (PBS containing 1% BSA) and incubated for 30min at RT. This was followed by washing of the wells three times with PBST (200µl). Then 50µl of donkey anti-goat-IgG HRP at a dilution of 1:1000 solution (santa cruz biotechnology) in PBS-BSA was added and incubated for 30min at RT. After three times of washing with PBST (200µl), the plate was developed plate by adding 50µl of a freshly prepared mix of 10ml 0.1M NaCitrate buffer at pH4.3, 5mg ABTS (Roche) and 10µl of 30% H₂O₂. The absorbance was read at 405nm.

### Example 6: Production of different Fc-fusion versions of miniFH

To increase the plasma half-life time of miniFH, the polypeptide of miniFH was fused to an Fc-part of an IgG antibody. Three different ways of connecting the miniFH to the Fc-part were realized (Fig. 1 B). First, the C-terminus of miniFH was fused to the N-terminus of the Fc-part i.e. miniFH-Fc. In a second construct the N-terminus of miniFH was fused to the C-terminus of the Fc-part, i.e. Fc-miniFH. And in a third construct also the N-terminus of miniFH was fused to the C-terminus of the Fc-part, but this time the Fc-part consisted of a shorter N-terminal sequence and hence lacks the disulfide dimerization domain of the heavy chain of an IgG, i.e. Fc-miniFH-short. However, due to very high affinity, non-covalent interactions between two identical CH3 domains, this construct remains a stable dimer under physiological conditions (which can e.g. be established by size-exclusion chromatography). This is in accordance with published reports (Ridgway et al. (1996), Protein Eng. 9(7):617-621; McAuley et al. (2008), Protein Sci. Publ. Protein Soc. 17(1):95-106). All three Fc-fusion variants of miniFH could be successfully produced recombinantly in the host Pichia pastoris and purified to a high degree of purity (Fig. 1C).

### Example 6: Fusion of miniFH to the N- or the C-terminus of an Fc-part results in similarly increased plasma half-life time

To evaluate how the Fc-fusion impacted on the plasma half-life time, about 0.1 mg of miniFH or one of the three Fc-fusion variants of miniFH were injected i.v. into mice and the plasma half-life time was determined by collecting plasma samples at several time points and analyzing the amount of protein present in these samples. Figure 2 shows that miniFH exhibits a beta-phase plasma half-life time of about 2.5 h while any of the Fc-fusion variants of miniFH have a dramatically increased beta phase plasma half-life time (T(1/2)) of about 20 h (miniFH-Fc T(1/2) = 23.1 h; Fc-miniFH T(1/2) = 21.1 h; Fc-miniFH-short (T(1/2) = 16.5 h). No substantial difference in plasma half-life time among the three different Fc-fusion version of miniFH could be determined.

### Example 7: Fusion of miniFH to the C-terminus of an Fc-part results in higher affinity for the complement activation product C3b

Since any of the Fc-fusion variants introduces dimerization and hence avidity for the binding partners of miniFH, it is expected that all Fc-fusion variants bind better to the main target of miniFH (i.e. C3b) than miniFH does itself. Since the driver of the expected higher affinity of miniFH for its target C3b is the introduction of avidity by dimerization, it was not anticipated that one Fc-fusion strategy results in better affinity than another Fc-fusion strategy. Figure 3 shows, however, that both Fc-miniFH fusion variants (Fc-miniFH and Fc-miniFH-short) bind substantially better to C3b in a surface plasmon resonance (SPR) experiment than miniFH-Fc, the variant with different Fc orientation. Thus Fc-miniFH and Fc-miniFH-short bind about threefold better to the main complement target C3b than miniFH-Fc does. For miniFH, which acted as a reference substance, the same affinity was determined as measured previously, cross-validating the SPR assay approach (Harder et al. 2016), J. Immunol. Baltim. Md 1950 196(2):866-876).

### Example 8: Fusion of miniFH to the C-terminus of an Fc-part also results in higher complement regulatory activity in human serum and hence in increased cell protection

Then it was investigated if the higher affinity for the main complement target C3b also results in enhanced complement regulatory activity. This was investigated in an haemolysis assay in which rabbit erythrocytes are lysed by the alternative pathway of complement when human serum is mixed with rabbit erythrocytes. MiniFH-Fc, Fc-miniFH and Fc-miniFH-short were tested in this assay alongside miniFH. Figure 4 shows that indeed the higher affinity of Fc-miniFH and Fc-miniFH-short for C3b resulted in higher complement regulatory activity when compared to miniFH-Fc. Also, miniFH-Fc exhibits a slightly increased (about twofold) affinity for C3b over miniFH due to avidity resulting from dimerization. But this slight increase does not precipitate in higher regulatory power towards the complement alternative pathway activity when compared to miniFH. The activities of miniFH and miniFH-Fc to regulate the complement cascade are very similar. In contrast the Fc-fusion of the Fc-miniFH orientation resulted in an about fourfold higher complement regulatory activity compared to miniFH (or miniFH-Fc). Hence, the untypical fusion of the miniFH N-terminus to the Fc-part C-terminus resulted not only in a higher affinity for the major complement target C3b, but also in pronounced higher overall complement regulatory activity in human serum.

Non-standard literature cited:
- Cereghino et al. (2000), FEMS Microbiol. Rev. 24(1):45-66.
- Harder et al. (2016), J. Immunol. Baltim. Md 1950. 196(2):866-876.
- Hillmen et al. (2006), NEJM355(12):1233
- McAuley et al. (2008), Protein Sci. Publ. Protein Soc. 17(1):95-106.
- Parkin & Cohen (2001) The Lancet 357: 1777-89.
- Ricklin et al. (2010) Nature Immunology 11: 785-797
- Ridgway et al. (1996), Protein Eng. 9(7):617-621.
- Romay-Penabad et al (2014), Lupus 23(12):1324
- Schmidt et al. (2008), Clin Exp Immunol. 151(1):14-24
- Schmidt et al. (2011), Protein Expr. Purif. 76(2):254-263.
- Schmidt et al. (2013), J. Immunol. 190(11):5712-5721.
- Schmidt et al. (2016). Immunobiology.221(4):503-511.
- WO 2013/142362 A1

## Claims

1. A multi-module polypeptide comprising
(i) an Fc receptor binding module;
(ii) a first complement control protein repeat (CCP) module; and
(iii) a second CCP module binding to at least one host cell surface marker, to complement factor C3b, to complement factor C4b, to a degradation product of complement factor C3b, and/or to a degradation product of complement factor C4b;
wherein said second CCP module is C-terminal of said Fc receptor binding module and of said first CCP module.

2. The multi-module polypeptide of claim 1, wherein said first CCP module (i) is a convertase decay accelerating module for convertases of the classical and/or alternative pathways of complement activation and/or (ii) is a binding module for complement factors C3b and/or C4b, preferably further having Factor I cofactor activity.

3. The multi-module polypeptide of claim 1 or 2, wherein said first CCP module comprises CCP domains 1 to 4 of a factor H, preferably human factor H; comprises CCP domains 1 to 3 of a complement receptor type 1 (CR1), preferably of human CR1; comprises CCP domains 1 to 4 of a decay accelerating factor (DAF), preferably human DAF, and/or comprises CCP domains 1 to 3 of a C4-binding protein (C4BP), preferably of human C4BP.

4. The multi-module polypeptide of any one of claims 1 to 3, wherein said first CCP module comprises, preferably consists of, an amino acid sequence as shown in SEQ ID NO:1 or an amino acid sequence being at least 70% identical thereto.

5. The multi-module polypeptide of any one of claims 1 to 4, wherein said second CCP module binds to polyanionic carbohydrates comprising sialic acids, glycosaminoglycans, and/or complement factor C3b or its degradation products.

6. The multi-module polypeptide of any one of claims 1 to 5, wherein said second CCP module comprises CCP domains 19 to 20 of factor H, preferably human factor H.

7. The multi-module polypeptide of any one of claims 1 to 6, wherein said second CCP module comprises, preferably consists of, an amino acid sequence as shown in SEQ ID NO:2 or an amino acid sequence being at least 70% identical thereto.

8. The multi-module polypeptide of any one of claims 1 to 7, wherein said Fc receptor binding module is an Fc module of an IgG, preferably of an IgG1.

9. The multi-module polypeptide of any one of claims 1 to 8, wherein said Fc receptor binding module comprises a peptide having an amino acid sequence of SEQ ID NO:3 or a sequence at least 70% identical thereto, preferably having an amino acid sequence of SEQ ID NO:3.

10. The multi-module polypeptide of any one of claims 1 to 9, wherein said first CCP module and said second CCP module together are comprised as a mini-FH comprising the amino acid sequence of one of SEQ ID NO:4, 5, 6, or 7 or a sequence at least 70% identical to at least one of said sequences, preferably comprising the amino acid sequence of one of SEQ ID NO:4, 5, 6, or 7.

11. The multi-module polypeptide of any one of claims 1 to 10, wherein said multi-module polypeptide comprises, preferably consists of, an amino acid sequence as shown in SEQ ID NO:8 or an amino acid sequence being at least 70% identical thereto.

12. A polynucleotide encoding a multi-module polypeptide of any one of claims 1 to 11.

13. A multi-module polypeptide according to any one of claims 1 to 11 and/or a polynucleotide according to claim 12 for use in medicine, preferably for treating and/or preventing inappropriate complement activation and/or a disease having inappropriate complement activation as a symptom.

14. The multi-module polypeptide and/or the polynucleotide for use of claim 13, wherein said disease having inappropriate complement activation as a symptom is ischemia reperfusion injury, antibody-mediated graft rejection, posttransplantation thrombotic microangiopathy, autoimmune hemolytic anemia, acute and delayed hemolytic transfusion reaction, cold agglutinin disease, rheumatoid arthritis, aquaporin-4-antibody-positive neuromyelitis optica, CD59-deficiency, C3-Glomerulopathy, atypical or typical hemolytic uremic syndrome, paroxysmal nocturnal hemoglobinuria, and/or age-related macular degeneration.

15. An in vitro method for preventing or reducing the degree of complement activation comprising applying a multi-module polypeptide according to any one of claims 1 to 11 to a reaction mixture, a tissue, and/or an organ comprising complement factors, thereby preventing or reducing the degree of complement activation in said reaction mixture, tissue, and/or organ.


SEQUENCE LISTING

<110>  Universität Ulm

<120>  Complement Inhibitors and Uses thereof

<130>  UU15304EP

<160>  16

<170>  PatentIn version 3.3

<210>  1
<211>  246
<212>  PRT
<213>  Artificial

<220>
<223>  CCP 1-4 of human Factor H

<400>  1

Glu Asp Cys Asn Glu Leu Pro Pro Arg Arg Asn Thr Glu Ile Leu Thr
1               5                   10                  15

Gly Ser Trp Ser Asp Gln Thr Tyr Pro Glu Gly Thr Gln Ala Ile Tyr
            20                  25                  30

Lys Cys Arg Pro Gly Tyr Arg Ser Leu Gly Asn Ile Ile Met Val Cys
        35                  40                  45

Arg Lys Gly Glu Trp Val Ala Leu Asn Pro Leu Arg Lys Cys Gln Lys
    50                  55                  60

Arg Pro Cys Gly His Pro Gly Asp Thr Pro Phe Gly Thr Phe Thr Leu
65                  70                  75                  80

Thr Gly Gly Asn Val Phe Glu Tyr Gly Val Lys Ala Val Tyr Thr Cys
                85                  90                  95

Asn Glu Gly Tyr Gln Leu Leu Gly Glu Ile Asn Tyr Arg Glu Cys Asp
            100                 105                 110

Thr Asp Gly Trp Thr Asn Asp Ile Pro Ile Cys Glu Val Val Lys Cys
        115                 120                 125

Leu Pro Val Thr Ala Pro Glu Asn Gly Lys Ile Val Ser Ser Ala Met
    130                 135                 140

Glu Pro Asp Arg Glu Tyr His Phe Gly Gln Ala Val Arg Phe Val Cys
145                 150                 155                 160

Asn Ser Gly Tyr Lys Ile Glu Gly Asp Glu Glu Met His Cys Ser Asp

165                 170                 175

Asp Gly Phe Trp Ser Lys Glu Lys Pro Lys Cys Val Glu Ile Ser Cys
            180                 185                 190

Lys Ser Pro Asp Val Ile Asn Gly Ser Pro Ile Ser Gln Lys Ile Ile
        195                 200                 205

Tyr Lys Glu Asn Glu Arg Phe Gln Tyr Lys Cys Asn Met Gly Tyr Glu
    210                 215                 220

Tyr Ser Glu Arg Gly Asp Ala Val Cys Thr Glu Ser Gly Trp Arg Pro
225                 230                 235                 240

Leu Pro Ser Cys Glu Glu
                245

<210>  2
<211>  124
<212>  PRT
<213>  Artificial

<220>
<223>  CCP 19 and 20 of human Factor H

<400>  2

Lys Cys Gly Pro Pro Pro Pro Ile Asp Asn Gly Asp Ile Thr Ser Phe
1               5                   10                  15

Pro Leu Ser Val Tyr Ala Pro Ala Ser Ser Val Glu Tyr Gln Cys Gln
            20                  25                  30

Asn Leu Tyr Gln Leu Glu Gly Asn Lys Arg Ile Thr Cys Arg Asn Gly
        35                  40                  45

Gln Trp Ser Glu Pro Pro Lys Cys Leu His Pro Cys Val Ile Ser Arg
    50                  55                  60

Glu Ile Met Glu Asn Tyr Asn Ile Ala Leu Arg Trp Thr Ala Lys Gln
65                  70                  75                  80

Lys Leu Tyr Ser Arg Thr Gly Glu Ser Val Glu Phe Val Cys Lys Arg
                85                  90                  95

Gly Tyr Arg Leu Ser Ser Arg Ser His Thr Leu Arg Thr Thr Cys Trp
            100                 105                 110

Asp Gly Lys Leu Glu Tyr Pro Thr Cys Ala Lys Arg
        115                 120

<210>  3
<211>  209
<212>  PRT
<213>  Artificial

<220>
<223>  Fc receptor binding module (short version)

<400>  3

Gly Gly Pro Ser Val Phe Leu Phe Pro Pro Lys Pro Lys Asp Thr Leu
1               5                   10                  15

Met Ile Ser Arg Thr Pro Glu Val Thr Cys Val Val Val Asp Val Ser
            20                  25                  30

His Glu Asp Pro Glu Val Lys Phe Asn Trp Tyr Val Asp Gly Val Glu
        35                  40                  45

Val His Asn Ala Lys Thr Lys Pro Arg Glu Glu Gln Tyr Gln Ser Thr
    50                  55                  60

Tyr Arg Val Val Ser Val Leu Thr Val Leu His Gln Asp Trp Leu Asn
65                  70                  75                  80

Gly Lys Glu Tyr Lys Cys Lys Val Ser Asn Lys Ala Leu Pro Ala Pro
                85                  90                  95

Ile Glu Lys Thr Ile Ser Lys Ala Lys Gly Gln Pro Arg Glu Pro Gln
            100                 105                 110

Val Tyr Thr Leu Pro Pro Ser Arg Glu Glu Met Thr Lys Asn Gln Val
        115                 120                 125

Ser Leu Thr Cys Leu Val Lys Gly Phe Tyr Pro Ser Asp Ile Ala Val
    130                 135                 140

Glu Trp Glu Ser Asn Gly Gln Pro Glu Asn Asn Tyr Lys Thr Thr Pro
145                 150                 155                 160

Pro Val Leu Asp Ser Asp Gly Ser Phe Phe Leu Tyr Ser Lys Leu Thr
                165                 170                 175

Val Asp Lys Ser Arg Trp Gln Gln Gly Asn Val Phe Ser Cys Ser Val
            180                 185                 190

Met His Glu Ala Leu His Asn His Tyr Thr Gln Lys Ser Leu Ser Leu
        195                 200                 205

Ser

<210>  4
<211>  384
<212>  PRT
<213>  Artificial

<220>
<223>  miniFH variant 1

<400>  4

Glu Asp Cys Asn Glu Leu Pro Pro Arg Arg Asn Thr Glu Ile Leu Thr
1               5                   10                  15

Gly Ser Trp Ser Asp Gln Thr Tyr Pro Glu Gly Thr Gln Ala Ile Tyr
            20                  25                  30

Lys Cys Arg Pro Gly Tyr Arg Ser Leu Gly Asn Ile Ile Met Val Cys
        35                  40                  45

Arg Lys Gly Glu Trp Val Ala Leu Asn Pro Leu Arg Lys Cys Gln Lys
    50                  55                  60

Arg Pro Cys Gly His Pro Gly Asp Thr Pro Phe Gly Thr Phe Thr Leu
65                  70                  75                  80

Thr Gly Gly Asn Val Phe Glu Tyr Gly Val Lys Ala Val Tyr Thr Cys
                85                  90                  95

Asn Glu Gly Tyr Gln Leu Leu Gly Glu Ile Asn Tyr Arg Glu Cys Asp
            100                 105                 110

Thr Asp Gly Trp Thr Asn Asp Ile Pro Ile Cys Glu Val Val Lys Cys
        115                 120                 125

Leu Pro Val Thr Ala Pro Glu Asn Gly Lys Ile Val Ser Ser Ala Met
    130                 135                 140

Glu Pro Asp Arg Glu Tyr His Phe Gly Gln Ala Val Arg Phe Val Cys
145                 150                 155                 160

Asn Ser Gly Tyr Lys Ile Glu Gly Asp Glu Glu Met His Cys Ser Asp
                165                 170                 175

Asp Gly Phe Trp Ser Lys Glu Lys Pro Lys Cys Val Glu Ile Ser Cys
            180                 185                 190

Lys Ser Pro Asp Val Ile Asn Gly Ser Pro Ile Ser Gln Lys Ile Ile
        195                 200                 205

Tyr Lys Glu Asn Glu Arg Phe Gln Tyr Lys Cys Asn Met Gly Tyr Glu
    210                 215                 220

Tyr Ser Glu Arg Gly Asp Ala Val Cys Thr Glu Ser Gly Trp Arg Pro
225                 230                 235                 240

Leu Pro Ser Cys Glu Glu Ala Gly Gly Gly Gly Gly Gly Gly Gly Gly
                245                 250                 255

Gly Gly Gly Gly Lys Cys Gly Pro Pro Pro Pro Ile Asp Asn Gly Asp
            260                 265                 270

Ile Thr Ser Phe Pro Leu Ser Val Tyr Ala Pro Ala Ser Ser Val Glu
        275                 280                 285

Tyr Gln Cys Gln Asn Leu Tyr Gln Leu Glu Gly Asn Lys Arg Ile Thr
    290                 295                 300

Cys Arg Asn Gly Gln Trp Ser Glu Pro Pro Lys Cys Leu His Pro Cys
305                 310                 315                 320

Val Ile Ser Arg Glu Ile Met Glu Asn Tyr Asn Ile Ala Leu Arg Trp
                325                 330                 335

Thr Ala Lys Gln Lys Leu Tyr Ser Arg Thr Gly Glu Ser Val Glu Phe
            340                 345                 350

Val Cys Lys Arg Gly Tyr Arg Leu Ser Ser Arg Ser His Thr Leu Arg
        355                 360                 365

Thr Thr Cys Trp Asp Gly Lys Leu Glu Tyr Pro Thr Cys Ala Lys Arg
    370                 375                 380

<210>  5
<211>  371
<212>  PRT
<213>  Artificial

<220>
<223>  miniFH variant 2

<400>  5

Glu Asp Cys Asn Glu Leu Pro Pro Arg Arg Asn Thr Glu Ile Leu Thr
1               5                   10                  15

Gly Ser Trp Ser Asp Gln Thr Tyr Pro Glu Gly Thr Gln Ala Ile Tyr

20                  25                  30

Lys Cys Arg Pro Gly Tyr Arg Ser Leu Gly Asn Ile Ile Met Val Cys
        35                  40                  45

Arg Lys Gly Glu Trp Val Ala Leu Asn Pro Leu Arg Lys Cys Gln Lys
    50                  55                  60

Arg Pro Cys Gly His Pro Gly Asp Thr Pro Phe Gly Thr Phe Thr Leu
65                  70                  75                  80

Thr Gly Gly Asn Val Phe Glu Tyr Gly Val Lys Ala Val Tyr Thr Cys
                85                  90                  95

Asn Glu Gly Tyr Gln Leu Leu Gly Glu Ile Asn Tyr Arg Glu Cys Asp
            100                 105                 110

Thr Asp Gly Trp Thr Asn Asp Ile Pro Ile Cys Glu Val Val Lys Cys
        115                 120                 125

Leu Pro Val Thr Ala Pro Glu Asn Gly Lys Ile Val Ser Ser Ala Met
    130                 135                 140

Glu Pro Asp Arg Glu Tyr His Phe Gly Gln Ala Val Arg Phe Val Cys
145                 150                 155                 160

Asn Ser Gly Tyr Lys Ile Glu Gly Asp Glu Glu Met His Cys Ser Asp
                165                 170                 175

Asp Gly Phe Trp Ser Lys Glu Lys Pro Lys Cys Val Glu Ile Ser Cys
            180                 185                 190

Lys Ser Pro Asp Val Ile Asn Gly Ser Pro Ile Ser Gln Lys Ile Ile
        195                 200                 205

Tyr Lys Glu Asn Glu Arg Phe Gln Tyr Lys Cys Asn Met Gly Tyr Glu
    210                 215                 220

Tyr Ser Glu Arg Gly Asp Ala Val Cys Thr Glu Ser Gly Trp Arg Pro
225                 230                 235                 240

Leu Pro Ser Cys Glu Glu Gly Lys Cys Gly Pro Pro Pro Pro Ile Asp
                245                 250                 255

Asn Gly Asp Ile Thr Ser Phe Pro Leu Ser Val Tyr Ala Pro Ala Ser
            260                 265                 270

Ser Val Glu Tyr Gln Cys Gln Asn Leu Tyr Gln Leu Glu Gly Asn Lys
        275                 280                 285

Arg Ile Thr Cys Arg Asn Gly Gln Trp Ser Glu Pro Pro Lys Cys Leu
    290                 295                 300

His Pro Cys Val Ile Ser Arg Glu Ile Met Glu Asn Tyr Asn Ile Ala
305                 310                 315                 320

Leu Arg Trp Thr Ala Lys Gln Lys Leu Tyr Ser Arg Thr Gly Glu Ser
                325                 330                 335

Val Glu Phe Val Cys Lys Arg Gly Tyr Arg Leu Ser Ser Arg Ser His
            340                 345                 350

Thr Leu Arg Thr Thr Cys Trp Asp Gly Lys Leu Glu Tyr Pro Thr Cys
        355                 360                 365

Ala Lys Arg
    370

<210>  6
<211>  379
<212>  PRT
<213>  Artificial

<220>
<223>  miniFH variant 3

<400>  6

Glu Asp Cys Asn Glu Leu Pro Pro Arg Arg Asn Thr Glu Ile Leu Thr
1               5                   10                  15

Gly Ser Trp Ser Asp Gln Thr Tyr Pro Glu Gly Thr Gln Ala Ile Tyr
            20                  25                  30

Lys Cys Arg Pro Gly Tyr Arg Ser Leu Gly Asn Ile Ile Met Val Cys
        35                  40                  45

Arg Lys Gly Glu Trp Val Ala Leu Asn Pro Leu Arg Lys Cys Gln Lys
    50                  55                  60

Arg Pro Cys Gly His Pro Gly Asp Thr Pro Phe Gly Thr Phe Thr Leu
65                  70                  75                  80

Thr Gly Gly Asn Val Phe Glu Tyr Gly Val Lys Ala Val Tyr Thr Cys
                85                  90                  95

Asn Glu Gly Tyr Gln Leu Leu Gly Glu Ile Asn Tyr Arg Glu Cys Asp

100                 105                 110

Thr Asp Gly Trp Thr Asn Asp Ile Pro Ile Cys Glu Val Val Lys Cys
        115                 120                 125

Leu Pro Val Thr Ala Pro Glu Asn Gly Lys Ile Val Ser Ser Ala Met
    130                 135                 140

Glu Pro Asp Arg Glu Tyr His Phe Gly Gln Ala Val Arg Phe Val Cys
145                 150                 155                 160

Asn Ser Gly Tyr Lys Ile Glu Gly Asp Glu Glu Met His Cys Ser Asp
                165                 170                 175

Asp Gly Phe Trp Ser Lys Glu Lys Pro Lys Cys Val Glu Ile Ser Cys
            180                 185                 190

Lys Ser Pro Asp Val Ile Asn Gly Ser Pro Ile Ser Gln Lys Ile Ile
        195                 200                 205

Tyr Lys Glu Asn Glu Arg Phe Gln Tyr Lys Cys Asn Met Gly Tyr Glu
    210                 215                 220

Tyr Ser Glu Arg Gly Asp Ala Val Cys Thr Glu Ser Gly Trp Arg Pro
225                 230                 235                 240

Leu Pro Ser Cys Glu Glu Ala Gly Gly Gly Gly Gly Gly Gly Gly Lys
                245                 250                 255

Cys Gly Pro Pro Pro Pro Ile Asp Asn Gly Asp Ile Thr Ser Phe Pro
            260                 265                 270

Leu Ser Val Tyr Ala Pro Ala Ser Ser Val Glu Tyr Gln Cys Gln Asn
        275                 280                 285

Leu Tyr Gln Leu Glu Gly Asn Lys Arg Ile Thr Cys Arg Asn Gly Gln
    290                 295                 300

Trp Ser Glu Pro Pro Lys Cys Leu His Pro Cys Val Ile Ser Arg Glu
305                 310                 315                 320

Ile Met Glu Asn Tyr Asn Ile Ala Leu Arg Trp Thr Ala Lys Gln Lys
                325                 330                 335

Leu Tyr Ser Arg Thr Gly Glu Ser Val Glu Phe Val Cys Lys Arg Gly
            340                 345                 350

Tyr Arg Leu Ser Ser Arg Ser His Thr Leu Arg Thr Thr Cys Trp Asp
        355                 360                 365

Gly Lys Leu Glu Tyr Pro Thr Cys Ala Lys Arg
    370                 375

<210>  7
<211>  376
<212>  PRT
<213>  Artificial

<220>
<223>  miniFH variant 4

<400>  7

Glu Asp Cys Asn Glu Leu Pro Pro Arg Arg Asn Thr Glu Ile Leu Thr
1               5                   10                  15

Gly Ser Trp Ser Asp Gln Thr Tyr Pro Glu Gly Thr Gln Ala Ile Tyr
            20                  25                  30

Lys Cys Arg Pro Gly Tyr Arg Ser Leu Gly Asn Ile Ile Met Val Cys
        35                  40                  45

Arg Lys Gly Glu Trp Val Ala Leu Asn Pro Leu Arg Lys Cys Gln Lys
    50                  55                  60

Arg Pro Cys Gly His Pro Gly Asp Thr Pro Phe Gly Thr Phe Thr Leu
65                  70                  75                  80

Thr Gly Gly Asn Val Phe Glu Tyr Gly Val Lys Ala Val Tyr Thr Cys
                85                  90                  95

Asn Glu Gly Tyr Gln Leu Leu Gly Glu Ile Asn Tyr Arg Glu Cys Asp
            100                 105                 110

Thr Asp Gly Trp Thr Asn Asp Ile Pro Ile Cys Glu Val Val Lys Cys
        115                 120                 125

Leu Pro Val Thr Ala Pro Glu Asn Gly Lys Ile Val Ser Ser Ala Met
    130                 135                 140

Glu Pro Asp Arg Glu Tyr His Phe Gly Gln Ala Val Arg Phe Val Cys
145                 150                 155                 160

Asn Ser Gly Tyr Lys Ile Glu Gly Asp Glu Glu Met His Cys Ser Asp
                165                 170                 175

Asp Gly Phe Trp Ser Lys Glu Lys Pro Lys Cys Val Glu Ile Ser Cys

180                 185                 190

Lys Ser Pro Asp Val Ile Asn Gly Ser Pro Ile Ser Gln Lys Ile Ile
        195                 200                 205

Tyr Lys Glu Asn Glu Arg Phe Gln Tyr Lys Cys Asn Met Gly Tyr Glu
    210                 215                 220

Tyr Ser Glu Arg Gly Asp Ala Val Cys Thr Glu Ser Gly Trp Arg Pro
225                 230                 235                 240

Leu Pro Ser Cys Glu Glu Ala Gly Gly Gly Gly Gly Lys Cys Gly Pro
                245                 250                 255

Pro Pro Pro Ile Asp Asn Gly Asp Ile Thr Ser Phe Pro Leu Ser Val
            260                 265                 270

Tyr Ala Pro Ala Ser Ser Val Glu Tyr Gln Cys Gln Asn Leu Tyr Gln
        275                 280                 285

Leu Glu Gly Asn Lys Arg Ile Thr Cys Arg Asn Gly Gln Trp Ser Glu
    290                 295                 300

Pro Pro Lys Cys Leu His Pro Cys Val Ile Ser Arg Glu Ile Met Glu
305                 310                 315                 320

Asn Tyr Asn Ile Ala Leu Arg Trp Thr Ala Lys Gln Lys Leu Tyr Ser
                325                 330                 335

Arg Thr Gly Glu Ser Val Glu Phe Val Cys Lys Arg Gly Tyr Arg Leu
            340                 345                 350

Ser Ser Arg Ser His Thr Leu Arg Thr Thr Cys Trp Asp Gly Lys Leu
        355                 360                 365

Glu Tyr Pro Thr Cys Ala Lys Arg
    370                 375

<210>  8
<211>  603
<212>  PRT
<213>  Artificial

<220>
<223>  FC-miniFH, short version

<400>  8

Gly Gly Pro Ser Val Phe Leu Phe Pro Pro Lys Pro Lys Asp Thr Leu
1               5                   10                  15

Met Ile Ser Arg Thr Pro Glu Val Thr Cys Val Val Val Asp Val Ser
            20                  25                  30

His Glu Asp Pro Glu Val Lys Phe Asn Trp Tyr Val Asp Gly Val Glu
        35                  40                  45

Val His Asn Ala Lys Thr Lys Pro Arg Glu Glu Gln Tyr Gln Ser Thr
    50                  55                  60

Tyr Arg Val Val Ser Val Leu Thr Val Leu His Gln Asp Trp Leu Asn
65                  70                  75                  80

Gly Lys Glu Tyr Lys Cys Lys Val Ser Asn Lys Ala Leu Pro Ala Pro
                85                  90                  95

Ile Glu Lys Thr Ile Ser Lys Ala Lys Gly Gln Pro Arg Glu Pro Gln
            100                 105                 110

Val Tyr Thr Leu Pro Pro Ser Arg Glu Glu Met Thr Lys Asn Gln Val
        115                 120                 125

Ser Leu Thr Cys Leu Val Lys Gly Phe Tyr Pro Ser Asp Ile Ala Val
    130                 135                 140

Glu Trp Glu Ser Asn Gly Gln Pro Glu Asn Asn Tyr Lys Thr Thr Pro
145                 150                 155                 160

Pro Val Leu Asp Ser Asp Gly Ser Phe Phe Leu Tyr Ser Lys Leu Thr
                165                 170                 175

Val Asp Lys Ser Arg Trp Gln Gln Gly Asn Val Phe Ser Cys Ser Val
            180                 185                 190

Met His Glu Ala Leu His Asn His Tyr Thr Gln Lys Ser Leu Ser Leu
        195                 200                 205

Ser Pro Gly Gly Gly Gly Gly Gly Gly Pro Gly Glu Asp Cys Asn Glu
    210                 215                 220

Leu Pro Pro Arg Arg Asn Thr Glu Ile Leu Thr Gly Ser Trp Ser Asp
225                 230                 235                 240

Gln Thr Tyr Pro Glu Gly Thr Gln Ala Ile Tyr Lys Cys Arg Pro Gly
                245                 250                 255

Tyr Arg Ser Leu Gly Asn Ile Ile Met Val Cys Arg Lys Gly Glu Trp

            260                 265                 270

Val Ala Leu Asn Pro Leu Arg Lys Cys Gln Lys Arg Pro Cys Gly His
        275                 280                 285

Pro Gly Asp Thr Pro Phe Gly Thr Phe Thr Leu Thr Gly Gly Asn Val
    290                 295                 300

Phe Glu Tyr Gly Val Lys Ala Val Tyr Thr Cys Asn Glu Gly Tyr Gln
305                 310                 315                 320

Leu Leu Gly Glu Ile Asn Tyr Arg Glu Cys Asp Thr Asp Gly Trp Thr
                325                 330                 335

Asn Asp Ile Pro Ile Cys Glu Val Val Lys Cys Leu Pro Val Thr Ala
            340                 345                 350

Pro Glu Asn Gly Lys Ile Val Ser Ser Ala Met Glu Pro Asp Arg Glu
        355                 360                 365

Tyr His Phe Gly Gln Ala Val Arg Phe Val Cys Asn Ser Gly Tyr Lys
    370                 375                 380

Ile Glu Gly Asp Glu Glu Met His Cys Ser Asp Asp Gly Phe Trp Ser
385                 390                 395                 400

Lys Glu Lys Pro Lys Cys Val Glu Ile Ser Cys Lys Ser Pro Asp Val
                405                 410                 415

Ile Asn Gly Ser Pro Ile Ser Gln Lys Ile Ile Tyr Lys Glu Asn Glu
            420                 425                 430

Arg Phe Gln Tyr Lys Cys Asn Met Gly Tyr Glu Tyr Ser Glu Arg Gly
        435                 440                 445

Asp Ala Val Cys Thr Glu Ser Gly Trp Arg Pro Leu Pro Ser Cys Glu
    450                 455                 460

Glu Ala Gly Gly Gly Gly Gly Gly Gly Gly Gly Gly Gly Gly Gly Lys
465                 470                 475                 480

Cys Gly Pro Pro Pro Pro Ile Asp Asn Gly Asp Ile Thr Ser Phe Pro
                485                 490                 495

Leu Ser Val Tyr Ala Pro Ala Ser Ser Val Glu Tyr Gln Cys Gln Asn
            500                 505                 510

Leu Tyr Gln Leu Glu Gly Asn Lys Arg Ile Thr Cys Arg Asn Gly Gln
        515                 520                 525

Trp Ser Glu Pro Pro Lys Cys Leu His Pro Cys Val Ile Ser Arg Glu
    530                 535                 540

Ile Met Glu Asn Tyr Asn Ile Ala Leu Arg Trp Thr Ala Lys Gln Lys
545                 550                 555                 560

Leu Tyr Ser Arg Thr Gly Glu Ser Val Glu Phe Val Cys Lys Arg Gly
                565                 570                 575

Tyr Arg Leu Ser Ser Arg Ser His Thr Leu Arg Thr Thr Cys Trp Asp
            580                 585                 590

Gly Lys Leu Glu Tyr Pro Thr Cys Ala Lys Arg
        595                 600

<210>  9
<211>  1827
<212>  DNA
<213>  Artificial

<220>
<223>  Fc-miniFH short, encoding

<400>  9
ggtggtccat ctgttttttt gttcccacca aagccaaagg acactttgat gatctccaga      60

actccagagg ttacttgtgt tgttgttgac gtttctcacg aggacccaga ggttaagttc     120

aactggtacg ttgacggtgt tgaggttcac aacgctaaga ctaagccaag agaggaacag     180

taccagtcca cttacagagt tgtttccgtt ttgactgtct tgcaccagga ctggttgaac     240

ggtaaagagt acaagtgtaa ggtttccaac aaggctttgc cagctccaat cgaaaagact     300

atctccaagg ctaagggtca gccaagagag ccacaagttt acactttgcc accatccaga     360

gaagagatga ctaagaacca ggtttccttg acttgtttgg ttaagggttt ctacccatcc     420

gacattgctg ttgaatggga gtctaacggt cagccagaga acaactacaa gactactcca     480

ccagttttgg actccgacgg ttcattcttc ttgtactcca agttgactgt tgacaagtcc     540

agatggcagc agggtaacgt tttctcctgt tctgttatgc acgaggctct tcacaaccac     600

tacactcaaa agtccttgtc cttgtctcca ggtggtggtg gtggaggtgg tcccggggag     660

gactgtaatg aattgccacc aagaagaaac actgagatct tgactggttc ctggtccgac     720

caaacttacc cagaaggtac tcaggctatc tacaagtgta gaccaggtta cagatccttg     780

ggtaacatca tcatggtctg tagaaagggt gagtgggttg ctttgaaccc attgagaaag     840

tgtcagaaaa gaccatgtgg tcacccaggt gatactccat tcggtacttt cactttgact     900

ggtggtaacg ttttcgagta cggtgttaag gctgtttaca cttgtaacga gggttaccag 960

ttgttgggtg agatcaacta cagagagtgt gacactgacg gttggactaa cgacatccca 1020

atctgtgagg ttgttaagtg tttgccagtt actgctccag agaacggtaa gatcgtttcc 1080

tctgctatgg aaccagacag agagtaccac ttcggtcagg ctgttagatt cgtctgtaac 1140

tccggttaca agatcgaggg tgacgaagaa atgcactgtt ctgatgacgg tttctggtcc 1200

aaagaaaagc caaagtgtgt tgagatctcc tgtaagtccc cagacgttat taacggttcc 1260

ccaatctccc agaagatcat ctacaaagag aacgagagat tccagtacaa gtgtaacatg 1320

ggttacgagt actccgagag aggtgacgct gtctgtactg aatctggttg gagaccattg 1380

ccatcctgtg aagaggccgg tggtggtgga ggtggaggtg gtggtggcgg aggtggtaaa 1440

tgtggtcccc caccaccaat tgacaacggt gacattactt ccttcccatt gtccgtttac 1500

gctccagctt cttccgttga gtaccagtgt cagaacttgt accagttgga gggtaacaag 1560

agaatcactt gtagaaacgg tcagtggtcc gagccaccaa aatgtctgca cccatgtgtt 1620

atctccagag aaatcatgga aaactacaac attgctttga gatggactgc taagcagaag 1680

ttgtactcca gaactggtga gtccgttgag ttcgtctgta agagaggtta cagattgtcc 1740

tccagatccc acactttgag aactacttgt tgggacggaa agcttgagta cccaacttgt 1800

gctaagagat aataatctag aacaaaa 1827

<210> 10
<211> 610
<212> PRT
<213> Artificial

<220>
<223> Fc-minFH long

<400> 10

Cys Pro Ala Pro Glu Leu Leu Gly Gly Pro Ser Val Phe Leu Phe Pro
1               5                   10                  15

Pro Lys Pro Lys Asp Thr Leu Met Ile Ser Arg Thr Pro Glu Val Thr
            20                  25                  30

Cys Val Val Val Asp Val Ser His Glu Asp Pro Glu Val Lys Phe Asn
        35                  40                  45

Trp Tyr Val Asp Gly Val Glu Val His Asn Ala Lys Thr Lys Pro Arg
    50                  55                  60

Glu Glu Gln Tyr Gln Ser Thr Tyr Arg Val Val Ser Val Leu Thr Val
65                  70                  75                  80

Leu His Gln Asp Trp Leu Asn Gly Lys Glu Tyr Lys Cys Lys Val Ser
                85                  90                  95

Asn Lys Ala Leu Pro Ala Pro Ile Glu Lys Thr Ile Ser Lys Ala Lys
            100                 105                 110

Gly Gln Pro Arg Glu Pro Gln Val Tyr Thr Leu Pro Pro Ser Arg Glu
        115                 120                 125

Glu Met Thr Lys Asn Gln Val Ser Leu Thr Cys Leu Val Lys Gly Phe
    130                 135                 140

Tyr Pro Ser Asp Ile Ala Val Glu Trp Glu Ser Asn Gly Gln Pro Glu
145                 150                 155                 160

Asn Asn Tyr Lys Thr Thr Pro Pro Val Leu Asp Ser Asp Gly Ser Phe
                165                 170                 175

Phe Leu Tyr Ser Lys Leu Thr Val Asp Lys Ser Arg Trp Gln Gln Gly
            180                 185                 190

Asn Val Phe Ser Cys Ser Val Met His Glu Ala Leu His Asn His Tyr
        195                 200                 205

Thr Gln Lys Ser Leu Ser Leu Ser Pro Gly Gly Gly Gly Gly Gly Gly
    210                 215                 220

Pro Gly Glu Asp Cys Asn Glu Leu Pro Pro Arg Arg Asn Thr Glu Ile
225                 230                 235                 240

Leu Thr Gly Ser Trp Ser Asp Gln Thr Tyr Pro Glu Gly Thr Gln Ala
                245                 250                 255

Ile Tyr Lys Cys Arg Pro Gly Tyr Arg Ser Leu Gly Asn Ile Ile Met
            260                 265                 270

Val Cys Arg Lys Gly Glu Trp Val Ala Leu Asn Pro Leu Arg Lys Cys
        275                 280                 285

Gln Lys Arg Pro Cys Gly His Pro Gly Asp Thr Pro Phe Gly Thr Phe
    290                 295                 300

Thr Leu Thr Gly Gly Asn Val Phe Glu Tyr Gly Val Lys Ala Val Tyr
305                 310                 315                 320

Thr Cys Asn Glu Gly Tyr Gln Leu Leu Gly Glu Ile Asn Tyr Arg Glu
                325                 330                 335

Cys Asp Thr Asp Gly Trp Thr Asn Asp Ile Pro Ile Cys Glu Val Val
            340                 345                 350

Lys Cys Leu Pro Val Thr Ala Pro Glu Asn Gly Lys Ile Val Ser Ser
        355                 360                 365

Ala Met Glu Pro Asp Arg Glu Tyr His Phe Gly Gln Ala Val Arg Phe
    370                 375                 380

Val Cys Asn Ser Gly Tyr Lys Ile Glu Gly Asp Glu Glu Met His Cys
385                 390                 395                 400

Ser Asp Asp Gly Phe Trp Ser Lys Glu Lys Pro Lys Cys Val Glu Ile
                405                 410                 415

Ser Cys Lys Ser Pro Asp Val Ile Asn Gly Ser Pro Ile Ser Gln Lys
            420                 425                 430

Ile Ile Tyr Lys Glu Asn Glu Arg Phe Gln Tyr Lys Cys Asn Met Gly
        435                 440                 445

Tyr Glu Tyr Ser Glu Arg Gly Asp Ala Val Cys Thr Glu Ser Gly Trp
    450                 455                 460

Arg Pro Leu Pro Ser Cys Glu Glu Ala Gly Gly Gly Gly Gly Gly Gly
465                 470                 475                 480

Gly Gly Gly Gly Gly Gly Lys Cys Gly Pro Pro Pro Pro Ile Asp Asn
                485                 490                 495

Gly Asp Ile Thr Ser Phe Pro Leu Ser Val Tyr Ala Pro Ala Ser Ser
            500                 505                 510

Val Glu Tyr Gln Cys Gln Asn Leu Tyr Gln Leu Glu Gly Asn Lys Arg
        515                 520                 525

Ile Thr Cys Arg Asn Gly Gln Trp Ser Glu Pro Pro Lys Cys Leu His
    530                 535                 540

Pro Cys Val Ile Ser Arg Glu Ile Met Glu Asn Tyr Asn Ile Ala Leu
545                 550                 555                 560

Arg Trp Thr Ala Lys Gln Lys Leu Tyr Ser Arg Thr Gly Glu Ser Val
                565                 570                 575

Glu Phe Val Cys Lys Arg Gly Tyr Arg Leu Ser Ser Arg Ser His Thr
            580                 585                 590

Leu Arg Thr Thr Cys Trp Asp Gly Lys Leu Glu Tyr Pro Thr Cys Ala
        595                 600                 605

Lys Arg
    610

<210>  11
<211>  1842
<212>  DNA
<213>  Artificial

<220>
<223>  Fc-miniFH long, encoding

<400>  11
tgtccagctc cagagttgtt gggtggtcca tctgtttttt tgttcccacc aaagccaaag      60

gacactttga tgatctccag aactccagag gttacttgtg ttgttgttga cgtttctcac     120

gaggacccag aggttaagtt caactggtac gttgacggtg ttgaggttca caacgctaag     180

actaagccaa gagaggaaca gtaccagtcc acttacagag ttgtttccgt tttgactgtc     240

ttgcaccagg actggttgaa cggtaaagag tacaagtgta aggtttccaa caaggctttg     300

ccagctccaa tcgaaaagac tatctccaag gctaagggtc agccaagaga gccacaagtt     360

tacactttgc caccatccag agaagagatg actaagaacc aggtttcctt gacttgtttg     420

gttaagggtt tctacccatc cgacattgct gttgaatggg agtctaacgg tcagccagag     480

aacaactaca agactactcc accagttttg gactccgacg gttcattctt cttgtactcc     540

aagttgactg ttgacaagtc cagatggcag cagggtaacg ttttctcctg ttctgttatg     600

cacgaggctc ttcacaacca ctacactcaa aagtccttgt ccttgtctcc aggtggtggt     660

ggtggaggtg gtcccgggga ggactgtaat gaattgccac caagaagaaa cactgagatc     720

ttgactggtt cctggtccga ccaaacttac ccagaaggta ctcaggctat ctacaagtgt     780

agaccaggtt acagatcctt gggtaacatc atcatggtct gtagaaaggg tgagtgggtt     840

gctttgaacc cattgagaaa gtgtcagaaa agaccatgtg gtcacccagg tgatactcca     900

ttcggtactt tcactttgac tggtggtaac gttttcgagt acggtgttaa ggctgtttac     960

acttgtaacg agggttacca gttgttgggt gagatcaact acagagagtg tgacactgac    1020

ggttggacta acgacatccc aatctgtgag gttgttaagt gtttgccagt tactgctcca    1080

gagaacggta agatcgtttc ctctgctatg gaaccagaca gagagtacca cttcggtcag    1140

gctgttagat tcgtctgtaa ctccggttac aagatcgagg gtgacgaaga aatgcactgt    1200

tctgatgacg gtttctggtc caaagaaaag ccaaagtgtg ttgagatctc ctgtaagtcc    1260

ccagacgtta ttaacggttc cccaatctcc cagaagatca tctacaaaga gaacgagaga    1320

ttccagtaca agtgtaacat gggttacgag tactccgaga gaggtgacgc tgtctgtact 1380

gaatctggtt ggagaccatt gccatcctgt gaagaggccg gtggtggtgg aggtggaggt 1440

ggtggtggcg gaggtggtaa atgtggtccc ccaccaccaa ttgacaacgg tgacattact 1500

tccttcccat tgtccgttta cgctccagct tcttccgttg agtaccagtg tcagaacttg 1560

taccagttgg agggtaacaa gagaatcact tgtagaaacg gtcagtggtc cgagccacca 1620

aaatgtctgc acccatgtgt tatctccaga gaaatcatgg aaaactacaa cattgctttg 1680

agatggactg ctaagcagaa gttgtactcc agaactggtg agtccgttga gttcgtctgt 1740

aagagaggtt acagattgtc ctccagatcc cacactttga gaactacttg ttgggacgga 1800

aagcttgagt acccaacttg tgctaagaga taataatcta ga 1842

<210> 12
<211> 216
<212> PRT
<213> Artificial

<220>
<223> Fc receptor binding module long

<400> 12

Cys Pro Ala Pro Glu Leu Leu Gly Gly Pro Ser Val Phe Leu Phe Pro
1               5                   10                  15

Pro Lys Pro Lys Asp Thr Leu Met Ile Ser Arg Thr Pro Glu Val Thr
            20                  25                  30

Cys Val Val Val Asp Val Ser His Glu Asp Pro Glu Val Lys Phe Asn
        35                  40                  45

Trp Tyr Val Asp Gly Val Glu Val His Asn Ala Lys Thr Lys Pro Arg
    50                  55                  60

Glu Glu Gln Tyr Gln Ser Thr Tyr Arg Val Val Ser Val Leu Thr Val
65                  70                  75                  80

Leu His Gln Asp Trp Leu Asn Gly Lys Glu Tyr Lys Cys Lys Val Ser
                85                  90                  95

Asn Lys Ala Leu Pro Ala Pro Ile Glu Lys Thr Ile Ser Lys Ala Lys
            100                 105                 110

Gly Gln Pro Arg Glu Pro Gln Val Tyr Thr Leu Pro Pro Ser Arg Glu
        115                 120                 125

Glu Met Thr Lys Asn Gln Val Ser Leu Thr Cys Leu Val Lys Gly Phe
    130                 135                 140

Tyr Pro Ser Asp Ile Ala Val Glu Trp Glu Ser Asn Gly Gln Pro Glu
145                 150                 155                 160

Asn Asn Tyr Lys Thr Thr Pro Pro Val Leu Asp Ser Asp Gly Ser Phe
                165                 170                 175

Phe Leu Tyr Ser Lys Leu Thr Val Asp Lys Ser Arg Trp Gln Gln Gly
            180                 185                 190

Asn Val Phe Ser Cys Ser Val Met His Glu Ala Leu His Asn His Tyr
        195                 200                 205

Thr Gln Lys Ser Leu Ser Leu Ser
    210                 215


<210>  13
<211>  615
<212>  PRT
<213>  Artificial

<220>
<223>  miniFH-Fc

<400>  13

Glu Asp Cys Asn Glu Leu Pro Pro Arg Arg Asn Thr Glu Ile Leu Thr
1               5                   10                  15

Gly Ser Trp Ser Asp Gln Thr Tyr Pro Glu Gly Thr Gln Ala Ile Tyr
            20                  25                  30

Lys Cys Arg Pro Gly Tyr Arg Ser Leu Gly Asn Ile Ile Met Val Cys
        35                  40                  45

Arg Lys Gly Glu Trp Val Ala Leu Asn Pro Leu Arg Lys Cys Gln Lys
    50                  55                  60

Arg Pro Cys Gly His Pro Gly Asp Thr Pro Phe Gly Thr Phe Thr Leu
65                  70                  75                  80

Thr Gly Gly Asn Val Phe Glu Tyr Gly Val Lys Ala Val Tyr Thr Cys
                85                  90                  95

Asn Glu Gly Tyr Gln Leu Leu Gly Glu Ile Asn Tyr Arg Glu Cys Asp
            100                 105                 110

Thr Asp Gly Trp Thr Asn Asp Ile Pro Ile Cys Glu Val Val Lys Cys
        115                 120                 125

Leu Pro Val Thr Ala Pro Glu Asn Gly Lys Ile Val Ser Ser Ala Met
    130                 135                 140

Glu Pro Asp Arg Glu Tyr His Phe Gly Gln Ala Val Arg Phe Val Cys
145                 150                 155                 160

Asn Ser Gly Tyr Lys Ile Glu Gly Asp Glu Glu Met His Cys Ser Asp
                165                 170                 175

Asp Gly Phe Trp Ser Lys Glu Lys Pro Lys Cys Val Glu Ile Ser Cys
            180                 185                 190

Lys Ser Pro Asp Val Ile Asn Gly Ser Pro Ile Ser Gln Lys Ile Ile
        195                 200                 205

Tyr Lys Glu Asn Glu Arg Phe Gln Tyr Lys Cys Asn Met Gly Tyr Glu
    210                 215                 220

Tyr Ser Glu Arg Gly Asp Ala Val Cys Thr Glu Ser Gly Trp Arg Pro
225                 230                 235                 240

Leu Pro Ser Cys Glu Glu Ala Gly Gly Gly Gly Gly Gly Gly Gly Gly
                245                 250                 255

Gly Gly Gly Gly Lys Cys Gly Pro Pro Pro Pro Ile Asp Asn Gly Asp
            260                 265                 270

Ile Thr Ser Phe Pro Leu Ser Val Tyr Ala Pro Ala Ser Ser Val Glu
        275                 280                 285

Tyr Gln Cys Gln Asn Leu Tyr Gln Leu Glu Gly Asn Lys Arg Ile Thr
    290                 295                 300

Cys Arg Asn Gly Gln Trp Ser Glu Pro Pro Lys Cys Leu His Pro Cys
305                 310                 315                 320

Val Ile Ser Arg Glu Ile Met Glu Asn Tyr Asn Ile Ala Leu Arg Trp
                325                 330                 335

Thr Ala Lys Gln Lys Leu Tyr Ser Arg Thr Gly Glu Ser Val Glu Phe
            340                 345                 350

Val Cys Lys Arg Gly Tyr Arg Leu Ser Ser Arg Ser His Thr Leu Arg
        355                 360                 365

Thr Thr Cys Trp Asp Gly Lys Leu Glu Tyr Pro Thr Cys Ala Gly Pro
    370                 375                 380

Gly Gly Gly Gly Gly Pro Gly Cys Pro Ala Pro Glu Leu Leu Gly Gly
385                 390                 395                 400

Pro Ser Val Phe Leu Phe Pro Pro Lys Pro Lys Asp Thr Leu Met Ile
                405                 410                 415

Ser Arg Thr Pro Glu Val Thr Cys Val Val Val Asp Val Ser His Glu
            420                 425                 430

Asp Pro Glu Val Lys Phe Asn Trp Tyr Val Asp Gly Val Glu Val His
        435                 440                 445

Asn Ala Lys Thr Lys Pro Arg Glu Glu Gln Tyr Gln Ser Thr Tyr Arg
    450                 455                 460

Val Val Ser Val Leu Thr Val Leu His Gln Asp Trp Leu Asn Gly Lys
465                 470                 475                 480

Glu Tyr Lys Cys Lys Val Ser Asn Lys Ala Leu Pro Ala Pro Ile Glu
                485                 490                 495

Lys Thr Ile Ser Lys Ala Lys Gly Gln Pro Arg Glu Pro Gln Val Tyr
            500                 505                 510

Thr Leu Pro Pro Ser Arg Glu Glu Met Thr Lys Asn Gln Val Ser Leu
        515                 520                 525

Thr Cys Leu Val Lys Gly Phe Tyr Pro Ser Asp Ile Ala Val Glu Trp
    530                 535                 540

Glu Ser Asn Gly Gln Pro Glu Asn Asn Tyr Lys Thr Thr Pro Pro Val
545                 550                 555                 560

Leu Asp Ser Asp Gly Ser Phe Phe Leu Tyr Ser Lys Leu Thr Val Asp
                565                 570                 575

Lys Ser Arg Trp Gln Gln Gly Asn Val Phe Ser Cys Ser Val Met His
            580                 585                 590

Glu Ala Leu His Asn His Tyr Thr Gln Lys Ser Leu Ser Leu Ser Pro
        595                 600                 605

Gly Gly Gly Gly Gly Gly Gly
    610                 615

<210>  14

<211>  6
<212>  PRT
<213>  Artificial

<220>
<223>  N-termminal extension 1

<400>  14

Glu Ala Glu Ala Ala Gly
1               5


<210>  15
<211>  4
<212>  PRT
<213>  Artificial

<220>
<223>  N-terminal extension 2

<400>  15

Glu Ala Ala Gly
1


<210>  16
<211>  2
<212>  PRT
<213>  Artificial

<220>
<223>  N-terminal extension 3

<400>  16

Ala Gly
1